# EUROPEAN PATENT SPECIFICATION

(11) **EP 4 114 466 B1**
(45) Date of publication and mention of the grant of the patent: **04.12.2024**
(21) Application number: 21715057.2
(22) Date of filing: 05.03.2021
(51) Int. Cl.: A61K 31/167, A61K 31/445, A61K 45/06, A61K 31/4422, A61P 9/00, A61P 9/10, A61P 25/04, A61P 9/08

(54) **TREATMENT OF PAIN AND VASOCONSTRICTION**
BEHANDLUNG VON SCHMERZEN UND GEFÄSSVERENGUNG
TRAITEMENT DE LA DOULEUR ET DE LA VASOCONSTRICTION

(30) Priority: 06.03.2020 US 202062986544 P; 21.04.2020 US 202063013468 P
(43) Date of publication of application: 11.01.2023
(73) Proprietor: Aisa Pharma, Inc., Boston, Massachusetts 02108 (US)
(72) Inventor: STERNLICHT, Andrew, Boston, Massachusetts 02108 (US)
(74) Representative: D Young & Co LLP
(86) International application number: PCT/US2021/021218
(87) International publication number: WO 2021/178903

(56) References cited:
- ENNIS HOLLY ET AL: "Calcium channel blockers for primary Raynaud's phenomenon", COCHRANE DATABASE OF SYSTEMATIC REVIEWS, 1 January 2016 (2016-01-01), XP055806271, Retrieved from the Internet <URL:https://www.ncbi.nlm.nih.gov/pmc/articles/PMC7065590/pdf/CD002069.pdf> DOI: 10.1002/14651858.CD002069.pub5
- KONNO YUUSUKE ET AL: "Vasodilatory Effect of Cilnidipine, an L-type and N-type Calcium Channel Blocker, on Rat Kidney Glomerular Arterioles Experimental Study", 1 November 2008 (2008-11-01), XP055807139, Retrieved from the Internet <URL:https://www.jstage.jst.go.jp/article/ihj/49/6/49_6_723/_pdf/-char/en> [retrieved on 20210525]
- MINAMI JUNICHI ET AL: "Effects of cilnidipine, a novel dihydropyridine calcium antagonist, on autonomic function, ambulatory blood pressure and heart rate in patients with essential hypertension : Cilnidipine in essential hypertension", BRITISH JOURNAL OF CLINICAL PHARMACOLOGY., vol. 50, no. 6, 1 December 2000 (2000-12-01), GB, pages 615 - 620, XP055807227, ISSN: 0306-5251, Retrieved from the Internet <URL:https://www.ncbi.nlm.nih.gov/pmc/articles/PMC2015014/pdf/bcp0050-0615.pdf> DOI: 10.1046/j.1365-2125.2000.00299.x
- CHANDRA K. SARAT ET AL: "The fourth-generation Calcium channel blocker: Cilnidipine", INDIAN HEART JOURNAL, vol. 65, no. 6, 1 December 2013 (2013-12-01), IN, pages 691 - 695, XP055807328, ISSN: 0019-4832, DOI: 10.1016/j.ihj.2013.11.001
- LEE SEUNGKYU ET AL: "Novel charged sodium and calcium channel inhibitor active against neurogenic inflammation", ELIFE, vol. 8, 25 November 2019 (2019-11-25), XP055807569, Retrieved from the Internet <URL:https://www.ncbi.nlm.nih.gov/pmc/articles/PMC6877086/pdf/elife-48118.pdf> DOI: 10.7554/eLife.48118
- EDWARD C EMERY ET AL: "Na v 1.7 and other voltage-gated sodium channels as drug targets for pain relief", EXPERT OPINION ON THERAPEUTIC TARGETS, vol. 20, no. 8, 12 April 2016 (2016-04-12), UK, pages 975 - 983, XP055437812, ISSN: 1472-8222, DOI: 10.1517/14728222.2016.1162295
- YAMAMOTO SHOHEI ET AL: "N- and L-type calcium channels blocker cilnidipine ameliorates neuropathic pain", EUROPEAN JOURNAL OF PHARMACOLOGY, ELSEVIER SCIENCE, NL, vol. 793, 5 November 2016 (2016-11-05), pages 66 - 75, XP029830234, ISSN: 0014-2999, DOI: 10.1016/J.EJPHAR.2016.11.001
- MA RENEE SIU YU ET AL: "Voltage gated sodium channels as therapeutic targets for chronic pain", JOURNAL OF PAIN RESEARCH, vol. Volume 12, 9 September 2019 (2019-09-09), pages 2709 - 2722, XP055807548, Retrieved from the Internet <URL:https://www.ncbi.nlm.nih.gov/pmc/articles/PMC6743634/pdf/jpr-12-2709.pdf> DOI: 10.2147/JPR.S207610

## Description

### CROSS-REFERENCE TO RELATED APPLICATION

This application claims priority to U.S. Provisional Application No. 62/986,544, filed on March 6, 2020; and U.S. Provisional Application No. 63/013,468, filed on April 21, 2020.

### TECHNICAL FIELD

This disclosure relates to cilnidipine or a pharmaceutically acceptable salt thereof, for use in treating Raynaud's syndrome in a subject in need thereof.

### BACKGROUND

Neuropathic pain can be caused by diseases and disorders affecting the somatosensory system. Neuropathic pain can be a debilitating symptom and consequence of a variety of ailments including diabetes, amputation, various malignant or benign cancers, infectious diseases, hereditary conditions, nerve damage from any cause, amongst others. Neuropathic pain can be associated or present with abnormal sensations such as allodynia (pain from non-painful stimuli), hyperesthesia (increased sensation to stimuli), or dysesthesia (abnormal sensation commonly perceived as either painful, itchy, burning, or restrictive), and may have continuous and/or episodic (e.g., paroxysmal) components.

In certain diseases and disorders, neuropathic pain accompanies dysregulation of blood flow (e.g., vasoconstriction). For example, Raynaud's syndrome is a medical condition that is characterized by episodic and periodic reductions of blood flow (referred to as "attacks") to, e.g., the extremities, causing pain, numbness, discoloration, burning sensation, and neuropathic pain. Discoloration of the skin whose blood supply is reduced and then reperfused can accompany these symptoms. Tissue ischemia from reduced blood flow, as well as reperfusion when vasoconstriction ceases, produces, painful burning sensations which can be experienced by the subject during the ischemic attack as well as when blood flow is reestablished. Symptoms of Raynaud's syndrome can be experienced after, e.g., changes in temperature (cold or hot) in body tissues and/or the experience of strong emotions (e.g., stress) by the subject. In some subjects who are severely affected, symptoms can progress to digital ulceration and/or gangrene with limb or digit loss.

Existing treatments of Raynaud's syndrome and/or treatment of neuropathic pain can include surgical treatment (sympathectomy), although rare, to decrease sympathetic activity in the affected limb(s). More common methods of treatment include administration of calcium channel blockers for their vasodilating effect, which are recommended as first line pharmacotherapy, and avoidance of triggering exposures (e.g., changes in temperature or emotional events). However, side effects of calcium channel blockers commonly occur with use and can include, e.g., constipation, nausea, headache, fatigue, rash, edema, pulmonary edema, drowsiness, dizziness, muscle weakness, muscle cramps, abnormal heartbeat, liver dysfunction, overgrowth of oral gums, flushing, low blood pressure, gastroesophageal reflux, bradycardia, tachycardia, QT interval prolongation, increased appetite, tenderness or bleeding of the gums, sexual dysfunction, abdominal pain, fainting, shortness of breath, altered taste, asthenia, muscle cramps, and itching. In some cases, administration of some types of calcium channel blockers can cause orthostatic hypotension. Other pharmacological approaches, including the use of phosphodiesterase inhibitors, prostanoids, angiotensin receptor blockers, endothelin receptor antagonists and topical nitroglycerin-containing creams have had marginal impact and often are accompanied by treatment-limiting side effects. These are considered second line treatment options, but a significant medical need still exists for an effective and safe pharmacological option for treatment for Raynaud's syndrome and the diseases in which it is included in the symptom complex of that disease.
Ennis Holly et al, Cochrane Database of Systematic Reviews, 2016, outlines a study to assess the effects of different calcium channel blockers for primary Raynaud's phenomenon as determined by attack rates, severity scores, participant-preference scores and physiological measurements.
Konno Yuusuke et al, Int. Heart J, 2008 outlines a study to assess the vasodilatory effect of cilnidipine on rat kidney glomerular arterioles.
Minami Junichi et al, British Journal of Clinical Pharmacology., 50, 2000, 615-620 describes the effects of cilnidipine on autonomic function, ambulatory blood pressure and heart rate in patients with essential hypertension.
Chandra K. Sarat et al, Indian Heart Journal, 65, 2013, 691-695 provides a review of antihypertensive drugs including cilnidipine.
Lee Seungkyu et al, ELIFE, 8, 2019 describes a cationic derivative of an N-type calcium channel-inhibitor to be applied extracellularly.
Edward C Emery et al, Expert Opinion on Therapeutic Targets, 20, 2016 describes Nav1.7 voltage-gated sodium channels as drug targets for pain relief.
Yamamoto Shohei et al, European Journal of Pharmacology, 793, 2016, 66-75 describes the use of cilnidipine for the treatment neuropathic pain.
Ma Renee Siu Yu et al, Journal of Pain Research, 12, 2019, 2709-2722 describes voltage gated sodium channels as therapeutic targets for chronic pain.

### SUMMARY

The present invention is as defined in the appended claims.

N-type calcium channels are localized, e.g., at the sympathetic pre-synaptic nerve terminals and play a role in the release of neurotransmitters such as gamma-aminobutyric acid (GABA), acetylcholine, dopamine, and norepinephrine. N-type calcium channels are known to regulate, e.g., neuronal excitability and the firing of action potentials in the neurons, which increases the transmission of neurotransmitters in nociceptive pathways. These neurotransmitters then bind to the receptors on the sensory neurons that cause a person to feel pain. The induction of neuropathic pain can, in certain cases, be a result of the redistribution and alteration of subunit compositions of sodium and calcium channels that can result in spontaneous firing at abnormal locations along the sensory pathway. This may result in unpleasant sensory perceptions including, for example, burning pain, a feeling of wetness, itching, electrical shock pain, and the sensation of pins and needles.

Neuropathic pain is notoriously difficult to treat, with only 40-60% of patients achieving a degree of relief after treatment. Existing drugs have the potential for addiction and/or can cause serious side effects that are, without wishing to be bound by theory, believed to be at least in part the result of unselective (e.g., non-discriminate or low selectivity) calcium channel inhibition.

Based on these considerations, N-selective dual N- and L-type calcium channel inhibition can be useful to treat diseases and disorders that are associated with dysregulation of blood flow and sympathetic nervous system overactivity, including those featuring symptoms of neuropathic pain.

A beneficial effect of L-type calcium channel inhibition is the dilation of the arteries in smooth muscle, causing an increase in arterial diameter, referred to as vasodilation. However, L-type calcium channel inhibition induces a homeostatic reflex mechanism in which norepinephrine is produced. The norepinephrine induces vasoconstriction, thus partially offsetting the vasodilating effects of the L-type calcium channel inhibition. A useful complementary effect of N-type calcium channel inhibition is the decrease of norepinephrine release and sympathetic outflow pre-synaptically in the spinal cord at the level of the dorsal root ganglion, which can counteract the homeostasis mechanism triggered by blockade of the L-type calcium channel. Disclosed herein are dual N-type and L-type calcium channel blockers selective for the N-type calcium channel (e.g., about 5-fold to 50-fold to about 100-fold selective) which can, for example, (1) reduce neuropathic pain, (2) induce vasodilation, and (3) counter the homeostatic vasoconstriction triggered by blockade of the L-type calcium channel. Dual N-type and L-type calcium channel blockers selective for the N-type calcium channel are therefore particularly effective at treating, e.g., Raynaud's disease.

Selective inhibition of the N-type calcium channel is, without wishing to be bound by theory, believed to result in reduced severity and/or frequency of side effects and increased tolerability compared to non-N-selective calcium channel blockade. Further, selective N-type CCBs may be effective for certain conditions at lower dosages and may provide higher efficacy relative to less selective calcium channel blockers. Additional advantages include an increase in bone density in certain subjects (e.g., subjects afflicted with osteoporosis) and beneficial renal effects. The beneficial renal effects are, without wishing to be bound by theory, believed to be an effect of reduced renal constriction, improvement in renal podocyte functioning, and improved blood flow in the kidney.

Additional advantages of selective N-channel blockade by CCBs, compared to CCBs that lack N-channel selectivity, can include:
- Improvement in endothelial function and endothelial concentrations of nitric oxide by improving blood flow, reducing pain that is, e.g., a consequence of reduced blood flow.
- Improvement in cardiac and left ventricle functioning resulting in reduction of pain due, e.g., to ischemia.
- Improvement in the incidence and severity of atherosclerosis including reducing pain caused, e.g., by a reduction in blood flow, and reducing the overall incidence of atherosclerotic-related events.
- Decrease in overall sympathetic nervous system activity and plasma concentration of norepinephrine, which can decrease pain due to net arteriole dilation and decrease in sympathetically mediated pain syndromes.
- Improvement in overall autonomic functioning, which may improve gut function in patients whose gut function (e.g., competency of the lower esophageal sphincter and peristalsis and gastric emptying), is compromised due to impaired autonomic function as occurs in certain disease states (e.g., scleroderma).

Selective N-type CCBs are also amenable to combination with other agents which may have an additive or complementary effect with the selective N-type CCB. For example, selective N-type CCBs may reduce the blood pressure of a hypertensive subject, which can be counterbalanced by combining the selective N-type CCB with an agent that increases blood pressure. Without wishing to be bound by theory, the selective N-type CCBs do not lower the blood pressure of a normotensive subject (i.e., a subject that does not have abnormal blood pressure; e.g., a subject that does not have hypertension). Further, the vasodilating effects of selective N-type CCBs can improve the effectiveness of an agent that treats erectile dysfunction. In addition, the vasodilating effects of selective N-type CCBs can improve the effectiveness of other agents used as second line treatment for patients having, e.g., Raynaud's disease and scleroderma. Cilnidipine exerts a balance of selective N- vs. L-type calcium channel inhibition (which can have a 5 fold to 50-fold to 100-fold selectivity for N-type calcium channel over L-type calcium channel), making it surprisingly effective at treating diseases and disorders characterized by neuropathic pain and vasoconstriction.

In one aspect, disclosed herein a compound which is cilnidipine or a pharmaceutically acceptable salt thereof, for use in treating Raynaud's syndrome in a subject in need thereof.

### Definitions

As used herein, the terms "about" and "approximately" are used interchangeably, and when used to refer to modify a numerical value, encompass a range of uncertainty of the numerical value of from 0% to 10% of the numerical value.

As used herein, the singular forms "a," "an," and "the" include plural referents unless the context clearly dictates otherwise.

As used herein, terms "treat" or "treatment" refer to therapeutic or palliative measures. Beneficial or desired clinical results include, but are not limited to, alleviation, in whole or in part, of symptoms associated with a disease or disorder or condition, diminishment of the extent of disease, stabilized (i.e., not worsening) state of disease, delay or slowing of disease progression, amelioration or palliation of the disease state (e.g., one or more symptoms of the disease), and remission (whether partial or total), whether detectable or undetectable. "Treatment" can also mean prolonging survival as compared to expected survival if not receiving treatment.

As used herein, the terms "subject" "individual," or "patient," are used interchangeably, refers to any animal, including mammals such as mice, rats, other rodents, rabbits, dogs, cats, swine, cattle, sheep, horses, primates, and humans. In some embodiments, the patient is a human. In some embodiments, the subject has experienced and/or exhibited at least one symptom of the disease or disorder to be treated and/or prevented. In some embodiments, the disease or disorder is associated with dysregulation of blood flow and sympathetic nervous system overactivity. In some embodiments, the disease or disorder is characterized by neuropathic pain, vasoconstriction, dysesthetic pain, burning pain, body temperature changes of the subject, hyperesthesia, changes in skin or tissue color, edema, changes in skin turgor, ruble, pallor, cyanosis, vasospasm, or any combination thereof.

As used herein, the phrase "dialysis support" refers to the assistance in maintaining concentrations of solutes (e.g., urea and/or creatinine) in a subject's blood within ranges that occur in subjects that have normal kidney function.

As used herein, the phrase "fixed dosage form" refers to the simultaneous administration of a dual N-type and L-type calcium channel blocker selective for the N-type calcium channel (cilnidipine in the present invention) and at least one additional therapeutic agent (e.g., a calcineurin inhibitor, a non-steroidal anti-inflammatory drug, or both to a subject in the form of a single composition or dosage.

As used herein, the phrase "dysregulation of blood flow and sympathetic nervous system overactivity" refers to reduced blood flow in any part of a subject's body and concomitant increased sympathetic outflow in the brainstem of the subject. In some embodiments, increased sympathetic outflow is evidenced by a decrease in heart function. In some embodiments, cardiac autonomic nervous function and thermographic (e.g., infrared thermographic) parameters can be assessed to indicate whether a subject exhibits dysregulation of blood flow and/or sympathetic nervous system overactivity. In certain embodiments, assessment of cardiac autonomic nervous function and thermographic parameters enables, e.g., a comparison of the function of the cardiac autonomic nervous system and the peripheral response to cold exposure. See clinical trial NCT 03094910 at clinicaltrials.gov/ct2/show/NCT03094910. In some embodiments, sympathetic nervous system overactivity can be assessed before and/or during a standardized mental arithmetic test. In certain embodiments, the mental arithmetic test can be conducted in a climate room. Parameters that can be measured during the mental arithmetic test include, but are not limited to, blood pressure, heart rate, forearm blood flow, fingertip laser Doppler flux, and/or venous concentrations of norepinephrine and epinephrine from the back of the hand. In some embodiments, measurement(s) of the foregoing parameters are compared to a control group that is not afflicted with dysregulation of blood flow and/or sympathetic nervous system overactivity. In some embodiments, indications of sympathetic nervous system overactivity include, but are not limited to, an increase in laser Doppler flux relative to the control group, a higher baseline diastolic blood pressure relative to the control group, a higher heart rate relative to the control group, a higher venous concentration of epinephrine relative to the control group, and/or higher a venous concentration of norepinephrine relative to the control group. See Int. Angiol., 1990, 9(2), 84-89.

As used herein, the term "dual N-type and L-type calcium channel blocker selective for the N-type calcium channel", "selective N-type calcium channel blocker", "selective N-type CCB", and "N-type selective CCB" refer to an agent that inhibits both N- and L-type calcium channels, and inhibits the N-type calcium channel to a greater degree than the L-type calcium channel. In some embodiments, the dual N-type and L-type calcium channel blocker selective for the N-type calcium channel has at least a 5-fold selectivity for the N-type calcium channel over the L-type calcium channel. For example, the dual N-type and L-type calcium channel blocker selective for the N-type calcium channel exhibits at least a 10-fold, at least a 30-fold, at least a 50-fold, at least a 80-fold, at least a 100-fold, at least a 300-fold, at least a 500-fold, at least a 800-fold, at least a 900-fold, or at least a 1000-fold selectivity for the N-type calcium channel over the L-type calcium channel. For example, the dual N-type and L-type calcium channel blocker selective for the N-type calcium channel exhibits at least a 50-fold to 100-fold selectivity for the N-type calcium channel over the L-type calcium channel. In the present invention, the dual N-type and L-type calcium channel blocker selective for the N-type calcium channel is cilnidipine or a pharmaceutically acceptable salt thereof

In the present invention, the dual N-type and L-type calcium channel blocker selective for the N-type calcium channel is also a Nav 1.7 sodium channel blocker (i.e., the dual N-type and L-type calcium channel blocker selective for the N-type calcium channel further inhibits a Nav 1.7 sodium channel). In the present invention, the dual N-type and L-type calcium channel blocker selective for the N-type calcium channel that further inhibits a sodium channel (e.g., Nav 1.7) is cilnidipine or a pharmaceutically acceptable salt thereof. As used herein, the term "non-N-type selective calcium channel blocker" refers to an agent that blocks one or more calcium channels, but either (1) does not block the N-type calcium channel, or (2) blocks the N-type calcium channel, but not selectively over the L-type calcium channel. Examples of non-N-type selective calcium channel blockers include, but are not limited to, nifedipine, nicardipine, amlodipine, Z-944, nimodipine, verapamil, diltiazem, felodipine, isradipine, nisoldipine, mibafredil, nilvidipine barnidipine, benidipine lacidipine, lercanidipine, manidipine and nitrendipine, and pharmaceutical salts thereof.

As used herein, the term "Nav 1.7 sodium channel blocker" or "Nav 1.7 sodium channel inhibitor" refers to an agent that can inhibit the Nav 1.7 sodium channel. In some embodiments, the Nav 1.7 sodium channel blocker inhibits the closed state of the Nav 1.7 sodium channel. In some embodiments, the Nav 1.7 sodium channel blocker inhibits the inactivated state of the Nav 1.7 sodium channel. In the present invention, the Nav 1.7 sodium channel blocker is also a dual N-type and L-type calcium channel blocker selective for the N-type calcium channel (i.e., the Nav 1.7 sodium channel blocker further inhibits the N-type and L-type calcium channels selectively for the N-type calcium channel). In the present invention, the Nav 1.7 sodium channel blocker that further inhibits the N-type and L-type calcium channels selectively for the N-type calcium channel is cilnidipine.

As used herein, the term "adverse effect" refers to an undesirable effect resulting from an alteration in normal physiology in a subject.

As used herein, the term "vasoconstriction" refers to the reduction in diameter of a blood vessel (e.g., an artery, vein, or capillary) resulting in reduced blood flow to the tissue the vasoconstricted blood vessels circulate blood to and from.

As used herein, the term "reducing susceptibility of a subject to cold-induced pain or discomfort" refers to reducing the pathologic response of a subject to experience pain or discomfort when subjected to an environment that lowers the temperature of a body part of the subject. In some embodiments, reducing susceptibility of a subject to cold-induced pain or discomfort can include reducing the likelihood that a subject will experience pain or discomfort when subjected to an environment that lowers the temperature of a body part of the subject. In some embodiments, reducing susceptibility of a subject to cold-induced pain or discomfort can include reducing the magnitude or intensity of pain or discomfort that a subject feels when subjected to an environment that lowers the temperature of a body part of the subject.

As used herein, the term "body temperature" refers to the temperature range of the body in a healthy, awake subject under normal conditions of thermoregulation as measured in the mouth, the rectum, the armpit, or the ear. For example, the temperature range in a healthy human subject under normal conditions of thermoregulation is 36.1 °C to 37.8 °C.

The term "therapeutically effective amount," as used herein, refers to a sufficient amount of a chemical entity (e.g., a dual N-type and L-type calcium channel blocker selective for the N-type calcium channel and/or a Nav 1.7 sodium channel blocker) being administered which will relieve to an extent one or more of the symptoms of the disease or condition being treated. The result includes reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case is determined using any suitable technique, such as a dose escalation study.

The term "pharmaceutically acceptable excipient" means a pharmaceutically-acceptable material, composition, or vehicle, such as a liquid or solid filler, diluent, carrier, solvent, or encapsulating material. In one embodiment, each component is " pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See, e.g.,* Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "pharmaceutically acceptable salt" may refer to pharmaceutically acceptable addition salts prepared from pharmaceutically acceptable non-toxic acids including inorganic and organic acids. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid and the like. The term "pharmaceutically acceptable salt" may also refer to pharmaceutically acceptable addition salts prepared by reacting a compound having an acidic group with a base to form a salt such as an ammonium salt, an alkali metal salt, such as a sodium or a potassium salt, an alkaline earth metal salt, such as a calcium or a magnesium salt, a salt of organic bases such as dicyclohexylamine, *N-*methyl-D-glucamine, tris(hydroxymethyl)methylamine, and salts with amino acids such as arginine, lysine, and the like, or by other methods previously determined. The pharmacologically acceptable salt is not specifically limited as far as it can be used in medicaments. Examples of a salt that the compounds described hereinform with a base include the following: salts thereof with inorganic bases such as sodium, potassium, magnesium, calcium, and aluminum; salts thereof with organic bases such as methylamine, ethylamine and ethanolamine; salts thereof with basic amino acids such as lysine and ornithine; and ammonium salt. The salts may be acid addition salts, which are specifically exemplified by acid addition salts with the following: mineral acids such as hydrochloric acid, hydrobromic acid, hydroiodic acid, sulfuric acid, nitric acid, and phosphoric acid: organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; acidic amino acids such as aspartic acid and glutamic acid.

The term "pharmaceutical composition" refers to a mixture of a compound described herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to: transdermal, intranasally, sublingual, intraspinal, or ocular administration.

The details of one or more embodiments of the invention are set forth in the description below. Other features and advantages of the invention will be apparent from the description and drawings, and from the claims.

### BRIEF DESCRIPTION OF THE DRAWINGS

FIG. 1 depicts a schematic diagram based on the modulated hypothesis of drug binding to various states of a sodium channel (NaCh).
FIG. 2 depicts a two pulse voltage protocol. The line marked with an asterisk indicates the voltage that was applied to the cell.
FIG. 3A is an overlay of inactivation current traces from VP1.
FIG. 3B depicts current traces at closed and inactivated states from the two pulse voltage protocol (VP2).
FIG. 4A depicts an It-plot for amlodipine at the closed state (P1).
FIG. 4B depicts an It-plot for amlodipine at the inactivated state (P2).
FIG. 5A depicts an It-plot for cilnidipine at the closed state (P1).
FIG. 5B is an It-plot for cilnidipine at the inactivated state (P2).
FIG. 6A depicts an It-plot for gabapentin at the closed state (P1).
FIG. 6B depicts an It-plot for gabapentin at the inactivated state (P2).
FIG. 7A depicts an example of an It-plot for bupivacaine at the closed state (P1).
FIG. 7B depicts an It-plot for bupivacaine at the inactivated state (P2).
FIG. 8A depicts a group hill fit for amlodipine at the closed state (P1).
FIG. 8B depicts a group hill fit for amlodipine at the inactivated state (P2).
FIG. 9A depicts a group hill fit for cilnidipine at the closed state (P1).
FIG. 9B depicts a group hill fit for cilnidipine at the inactivated state (P2).
FIG. 10A depicts a group hill fit for gabapentin at the closed state (P1).
FIG. 10B depicts a group hill fit for gabapentin at the (P2) inactivated state.
FIG. 11A depicts a group hill fit for bupivacaine at closed state (P1).
FIG. 11B depicts a group hill fit for bupivacaine at (P2) inactivated state.
FIG. 12 depicts percent Nav 1.7 sodium channel inhibition at Cₘₐₓ for nifedipine 40 mg, cilnidipine 10 mg, cilnidipine 20 mg, amlodipine 10 mg, gabapentin 1800 mg, and bupivacaine 1 µM.

### DETAILED DESCRIPTION

In one aspect, disclosed herein is a compound which is cilnidipine or a pharmaceutically acceptable salt thereof, for use in treating Raynaud's syndrome in a subject in need thereof.

Examples of N-type calcium channels include, but are not limited to, the Cav2.2 Type, which has two subunits, Cav 2.2a and Cav2.2b, both of which have an alpha 1 subunit of 2.2 and are affected by N type current.

In some embodiments, the therapeutically effective amount of cilnidipine or a pharmaceutically acceptable salt thereof is reduced compared to the therapeutically effective amount of a non-N-selective calcium channel blocker useful to treat the disease or disorder.

In some embodiments, one or more side effects experienced by the subject after administration of the cilnidipine or a pharmaceutically acceptable salt thereof are less severe or less frequent than as compared to the side effects experienced by a subject after administration of a therapeutically effective amount of a non-N-selective calcium channel blocker useful to treat the disease or disorder. Without wishing to be bound by theory, this may allow a higher dose of the cilnidipine or a pharmaceutically acceptable salt thereof to be administered to the subject, which can, e.g., result in a higher treatment efficacy than the non-N-selective calcium channel blocker.

In some embodiments, the cilnidipine or a pharmaceutically acceptable salt thereof is more effective than the non-N-selective calcium channel blocker in treating an adverse effect of the disease or disorder. In some embodiments, the adverse effect is a symptom or a clinical manifestation of the disease or disorder.

It has been shown that dual N-type and L-type calcium channel blockers selective for the N-type calcium channels have fewer and less severe side effects, better tolerability, and are safer than less-N-selective calcium channel blockers. It is believed that this is due to the increased inhibition of the N channel relative to the L channel. By decreasing sympathetic activity, as well as by dilating not only arterioles but the venous system, dual N-type and L-type calcium channel blockers selective for the N-type calcium channel appear to be associated with less adverse events in patients treated for hypertension than patients treated with dual L and N- calcium channel antagonists with lower levels of N - selectivity.

The disease or disorder to be treated is Raynaud's syndrome. For example, the Raynaud's syndrome is selected from the group consisting of: primary Raynaud's syndrome; secondary Raynaud's syndrome; Raynaud's syndrome of the nipple, nose, ear, penis, tongue, and/or any alar circulatory region. For example, the Raynaud's syndrome is secondary Raynaud's syndrome.

In some embodiments, the non-N-selective calcium channel blocker is a dihydropyridine. In some other embodiments, the non-N-selective calcium channel blocker is a non-dihydropyridine. Non-limiting examples of non-N-selective calcium channel blockers include, but are not limited to: nifedipine, nicardipine, amlodipine, Z-944, nimodipine, verapamil, diltiazem, felodipine, isradipine, nisoldipine, mibafredil, nilvidipine barnidipine, benidipine lacidipine, lercanidipine, manidipine and nitrendipine, and pharmaceutically acceptable salts thereof.

In some embodiments, the therapeutically effective amount of the cilnidipine or a pharmaceutically acceptable salt thereof is at least 10% lower than the therapeutically effective amount of the non-N-selective calcium channel blocker. For example, the therapeutically effective amount of the cilnidipine or a pharmaceutically acceptable salt thereof is at least 15% lower, at least 20% lower, at least 25% lower, at least 30% lower, at least 35% lower, at least 40% lower, at least 45% lower, at least 50% lower, at least 55% lower, at least 60% lower, at least 65% lower, at least 70% lower, at least 75% lower, at least 80% lower, at least 85% lower, at least 90% lower, or at least 95% lower than the therapeutically effective amount of the non-N-selective calcium channel blocker.

In some embodiments, the therapeutically effective amount of the cilnidipine or a pharmaceutically acceptable salt thereof decreases the blood pressure (e.g., the systolic blood pressure) of the subject to a lesser degree than the therapeutically effective amount of the non-N-selective calcium channel blocker. In some embodiments, the cilnidipine or a pharmaceutically acceptable salt thereof decreases the blood pressure (e.g., the systolic blood pressure) of the subject at least 5% less than the non-N-selective calcium channel blocker. For example, the cilnidipine or a pharmaceutically acceptable salt thereof decreases the blood pressure (e.g., the systolic blood pressure) of the subject at least 10% less, at least 15% less, at least 20% less, at least 25% less, at least 30% less, at least 35% less, at least 40% less, at least 45% less, at least 50% less, at least 55% less, at least 60% less, at least 65% less, at least 70% less, at least 75% less, at least 80% less, at least 85% less, at least 90% less, or at least 95% less, than the non-N-selective calcium channel blocker.

In some embodiments, the side effects are selected from the group consisting of: constipation, nausea, headache, fatigue, rash, edema, pulmonary edema, peripheral edema, heart rate changes, drowsiness, dizziness, muscle weakness, muscle cramps, abnormal heartbeat, liver dysfunction, overgrowth of oral gums, flushing, low blood pressure, gastroesophageal reflux, bradycardia, tachycardia, QT interval prolongation, increased appetite, tenderness or bleeding of the gums, sexual dysfunction, abdominal pain, fainting, shortness of breath, altered taste, asthenia, muscle cramps, and itching.

In some embodiments, the subject is also diagnosed with hypertension; and wherein after administration of the cilnidipine or a pharmaceutically acceptable salt thereof, the blood pressure (e.g., the systolic blood pressure) of the subject is reduced.

In some embodiments, the subject has hypertension; and wherein after administration of the cilnidipine or a pharmaceutically acceptable salt thereof to the subject, the blood pressure (e.g., the systolic blood pressure) of the subject is reduced.

In some embodiments, the subject is being treated for hypertension; and wherein after administration of the cilnidipine or a pharmaceutically acceptable salt thereof to the subject, the blood pressure (e.g., the systolic blood pressure) of the subject is reduced.

In some embodiments, the subject also has hypertension; and wherein after administration of the cilnidipine or a pharmaceutically acceptable salt thereof to the subject, the blood pressure (e.g., the systolic blood pressure) of the subject is reduced. In some embodiments, the subject does not have hypertension; and wherein after administration of the cilnidipine or a pharmaceutically acceptable salt thereof to the subject, the blood pressure (e.g., the systolic blood pressure) of the subject is not reduced. Without wishing to be bound by theory, it is believed that when the subject has hypertension, the cilnidipine or a pharmaceutically acceptable salt thereof reduces the blood pressure of the subject; however, when the subject does not have hypertension (i.e., the subject is normotensive), the cilnidipine or a pharmaceutically acceptable salt thereof does not reduce the blood pressure of the subject. In some embodiments, after administration of the cilnidipine or a pharmaceutically acceptable salt thereof to the subject, the cardiac function of the subject is improved (e.g., left ventricular function of the subject is improved).

In some embodiments, after administration of the cilnidipine or a pharmaceutically acceptable salt thereof to the subject, the cardiac function of the subject is improved. In some embodiments, improving the cardiac function in the subject comprises improving the left ventricular function of the subject. In some embodiments, the subject has hypertension. In some embodiments, the subject does not have hypertension.

In some embodiments, the subject has hypertension; and after administration of the cilnidipine or a pharmaceutically acceptable salt thereof to the subject, the blood pressure of the subject is decreased and the cardiac function (e.g., left ventricular function) of the subject is improved.

In some embodiments, the subject does not have hypertension; and after administration of the cilnidipine or a pharmaceutically acceptable salt thereof to the subject, the blood pressure of the subject is not decreased and the cardiac function (e.g., left ventricular function) of the subject is improved.

In some embodiments, the subject has hypertension and osteoporosis; and after administration of the cilnidipine or a pharmaceutically acceptable salt thereof to the subject, the blood pressure of the subject is decreased and the bone density in the subject is increased.

In some embodiments, the subject does not have hypertension; the subject has osteoporosis; and after administration of the cilnidipine or a pharmaceutically acceptable salt thereof to the subject, the blood pressure of the subject is not decreased and the bone density in the subject is increased.

In some embodiments, the subject has hypertension; and after administration of the cilnidipine or a pharmaceutically acceptable salt thereof to the subject, the blood pressure of the subject is decreased and the bone density in the subject is increased.

In some embodiments, the subject does not have hypertension; and after administration of the cilnidipine or a pharmaceutically acceptable salt thereof to the subject, the blood pressure of the subject is not decreased and the bone density in the subject is increased.

In some embodiments, the subject has hypertension and atherosclerosis; and after administration of the cilnidipine or a pharmaceutically acceptable salt thereof to the subject, the blood pressure of the subject is decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject does not have hypertension; the subject has atherosclerosis; and after administration of the cilnidipine or a pharmaceutically acceptable salt thereof to the subject, the blood pressure of the subject is not decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject has hypertension; and after administration of the cilnidipine or a pharmaceutically acceptable salt thereof to the subject, the blood pressure of the subject is decreased and renal function in the subject is improved.

In some embodiments, the subject does not have hypertension; and after administration of the cilnidipine or a pharmaceutically acceptable salt thereof to the subject, the blood pressure of the subject is not decreased and renal function in the subject is improved.

In some embodiments, the subject has hypertension; and after administration of the cilnidipine or a pharmaceutically acceptable salt thereof to the subject, the blood pressure of the subject is decreased and renal function in the subject is improved. In some embodiments, the subject does not have hypertension; and after administration of the cilnidipine or a pharmaceutically acceptable salt thereof to the subject, the blood pressure of the subject is not decreased and renal function in the subject is improved.

In some embodiments, the subject has hypertension; the subject was previously treated with antihypertensive agents before administration of the cilnidipine or a pharmaceutically acceptable salt thereof; and after administration of the cilnidipine or a pharmaceutically acceptable salt thereof to the subject, the blood pressure of the subject is decreased.

In some embodiments, the subject does not have hypertension; the subject was previously treated with antihypertensive agents before administration of the cilnidipine or a pharmaceutically acceptable salt thereof; and after administration of the cilnidipine or a pharmaceutically acceptable salt thereof to the subject, the blood pressure of the subject is is not decreased.

In some embodiments, the subject has hypertension; the subject has scleroderma; the subject has a digital ulcer; and after administration of the cilnidipine or a pharmaceutically acceptable salt thereof to the subject, the blood pressure of the subject is decreased, and the digital ulcer is treated. In some embodiments, treating the digital ulcer comprises healing or improving the condition of the digital ulcer.

In some embodiments, the subject does not have hypertension; the subject has scleroderma; the subject has a digital ulcer; and after administration of the cilnidipine or a pharmaceutically acceptable salt thereof to the subject, the blood pressure of the subject is not reduced, and the digital ulcer is treated. In some embodiments, treating the digital ulcer comprises healing or improving the condition of the digital ulcer.

In some embodiments, the subject has hypertension; the subject has scleroderma; the disease or disorder associated with dysregulation of blood flow and sympathetic nervous system overactivity is Raynaud's syndrome; and after administration of the cilnidipine or a pharmaceutically acceptable salt thereof to the subject, the blood pressure of the subject is decreased.

In some embodiments, the subject does not have hypertension; the subject has scleroderma; the disease or disorder associated with dysregulation of blood flow and sympathetic nervous system overactivity is Raynaud's syndrome; and after administration of the cilnidipine or a pharmaceutically acceptable salt thereof, the blood pressure of the subject is not reduced.

In some embodiments, the blood pressure (e.g., systolic blood pressure) of the subject is reduced by greater than 1 mm Hg. For example, the systolic blood pressure of the subject is reduced by greater than 2, 5, 8, 10, 12, 15, 20, 25, or 30 mm Hg. For example, the systolic blood pressure of the subject is reduced by about 1-5 mm Hg, about 5-10 mm Hg, about 10-15 mm Hg, about 15-20 mm Hg, or about 20-30 mm Hg.

In some embodiments, the subject has atherosclerosis; and after administration of the cilnidipine or a pharmaceutically acceptable salt thereof to the subject, the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject has a digital ulcer; and after administration of the cilnidipine or a pharmaceutically acceptable salt thereof to the subject, the digital ulcer is treated. In some embodiments, treating the digital ulcer comprises healing or improving the condition of the digital ulcer.

In some embodiments, the subject is also being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject has lupus, scleroderma, scleroderma with interstitial lung disease, rheumatoid arthritis, atherosclerosis, cryoglobulinemia, polycythemia, dermatomyositis, polymyositis, Sjögren's syndrome, or any combination thereof. In some embodiments, the subject is diagnosed with lupus, scleroderma, scleroderma with interstitial lung disease, rheumatoid arthritis, atherosclerosis, cryoglobulinemia, polycythemia, dermatomyositis, polymyositis, Sjögren's syndrome, or any combination thereof. In some embodiments, the treatment for lupus, scleroderma, scleroderma with interstitial lung disease, rheumatoid arthritis, atherosclerosis, cryoglobulinemia, polycythemia, dermatomyositis, polymyositis, Sjögren's syndrome, or any combination thereof comprises administering a therapeutic agent. Therapeutic agents known in the art for treating lupus, scleroderma, scleroderma with interstitial lung disease, rheumatoid arthritis, atherosclerosis, cryoglobulinemia, polycythemia, dermatomyositis, polymyositis, and Sjögren's syndrome can be found in, e.g., the Physicians' desk reference. (71st ed.). (2017). Montvale, NJ: PDR Network.

In certain embodiments, the subject is being treated for scleroderma. For example, the subject is being treated for scleroderma with interstitial lung disease. In certain embodiments, the subject has scleroderma. For example, the subject has scleroderma with interstitial lung disease. In certain embodiments, the subject is diagnosed with scleroderma. For example, the subject is diagnosed with scleroderma with interstitial lung disease. In some embodiments, the cilnidipine or a pharmaceutically acceptable salt thereof is administered with an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof. For example, cilnidipine or a pharmaceutically acceptable salt thereof is administered with nintedanib. In some embodiments, the cilnidipine or a pharmaceutically acceptable salt thereof is administered with a calcineurin inhibitor, a non-steroidal anti-inflammatory drug, or both. In some embodiments, the cilnidipine or a pharmaceutically acceptable salt thereof, the calcineurin inhibitor, and the non-steroidal anti-inflammatory drug are administered separately, sequentially, or simultaneously. In some embodiments, the cilnidipine or a pharmaceutically acceptable salt thereof, the calcineurin inhibitor, and the non-steroidal anti-inflammatory drug are administered separately. In some embodiments, the cilnidipine or a pharmaceutically acceptable salt thereof, the calcineurin inhibitor, and the non-steroidal anti-inflammatory drug are administered sequentially. In some embodiments, the cilnidipine or a pharmaceutically acceptable salt thereof, the calcineurin inhibitor, and the non-steroidal anti-inflammatory drug are administered simultaneously. In some embodiments, the cilnidipine or a pharmaceutically acceptable salt thereof, the calcineurin inhibitor, and the non-steroidal anti-inflammatory drug are administered simultaneously as a fixed dosage form. In some embodiments, the calcineurin inhibitor is a cyclosporine. In some embodiments, the non-steroidal anti-inflammatory drug is aspirin.

In certain embodiments, the subject is being treated for lupus. In certain embodiments, the subject is diagnosed with lupus. In certain embodiments, the subject has lupus. In some embodiments, the cilnidipine or a pharmaceutically acceptable salt thereof is administered with an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In certain embodiments, the subject is being treated for rheumatoid arthritis. In certain embodiments, the subject has rheumatoid arthritis. In certain embodiments, the subject is diagnosed with rheumatoid arthritis. In some embodiments, the cilnidipine or a pharmaceutically acceptable salt thereof is administered with an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In certain embodiments, the subject is being treated for Sjögren's syndrome. In certain embodiments, the subject has Sjögren's syndrome. In certain embodiments, the subject is diagnosed with Sjögren's syndrome. In some embodiments, the cilnidipine or a pharmaceutically acceptable salt thereof is administered with an agent selected from the group consisting of: plaquenil, an antimalarial drug (e.g., hydroxychloroquine), evoxac, cevimeline, infliximab, or any combination thereof.

In certain embodiments, the subject is being treated for idiopathic pulmonary fibrosis. In certain embodiments, the subject has idiopathic pulmonary fibrosis. In certain embodiments, the subject is diagnosed with idiopathic pulmonary fibrosis. In some embodiments, the cilnidipine or a pharmaceutically acceptable salt thereof is administered with an agent selected from the group consisting of: nintedanib, pirfenidone, or any combination thereof.

In some embodiments, the disease or disorder associated with dysregulation of blood flow and sympathetic nervous system overactivity in a subject is secondary Raynaud's disease; the subject is also being treated for scleroderma; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder associated with dysregulation of blood flow and sympathetic nervous system overactivity in a subject is secondary Raynaud's disease; the subject is also being treated for scleroderma with interstitial lung disease; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder associated with dysregulation of blood flow and sympathetic nervous system overactivity in a subject is secondary Raynaud's disease; the subject is also being treated for lupus; the dual N-type and L-type calcium channel blocker selective for the N-type calcium channel is cilnidipine; and the subject is further administered an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In some embodiments, the disease or disorder associated with dysregulation of blood flow and sympathetic nervous system overactivity in a subject is secondary Raynaud's disease; the subject is also being treated for rheumatoid arthritis; the dual N-type and L-type calcium channel blocker selective for the N-type calcium channel is cilnidipine; and the subject is further administered an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In some embodiments, the disease or disorder associated with dysregulation of blood flow and sympathetic nervous system overactivity in a subject is secondary Raynaud's disease; the subject is also being treated for Sjögren's syndrome; the dual N-type and L-type calcium channel blocker selective for the N-type calcium channel is cilnidipine; and the subject is further administered an agent selected from the group consisting of: plaquenil, an antimalarial drug (e.g., hydroxychloroquine), evoxac, cevimeline, infliximab, or any combination thereof.

In some embodiments, the disease or disorder associated with dysregulation of blood flow and sympathetic nervous system overactivity in a subject is secondary Raynaud's disease; the subject is also being treated for idiopathic pulmonary fibrosis; the dual N-type and L-type calcium channel blocker selective for the N-type calcium channel is cilnidipine; and the subject is further administered an agent selected from the group consisting of: nintedanib, pirfenidone, or any combination thereof.

In some embodiments, the adverse effect is pain (e.g., neuropathic pain or migraine (e.g., congenital migraine)), cerebellar ataxia, angina, epilepsy, hypertension, ischemia, and arrhythmia. In certain embodiments, the pain is neuropathic pain. For example, the neuropathic pain is burning pain, a feeling of wetness, pruritis (itching), electrical shock pain, the sensation of pins and needles, a lancinating pain, a dull pain, or a crampy pain. In certain embodiments the pain is a lancinating pain, a dull pain, or a crampy pain. In some embodiments, the adverse effect is numbness, sedation, decreased respiratory rate, constipation, disorientation, tachycardia, hyperkinetic movements, addiction (e.g., alcohol addiction), and inflammation.

In some embodiments, the adverse effect is vasoconstriction. In certain embodiments, the vasoconstriction comprises vasoconstriction of a body part, and the temperature of the vasoconstricted body part is lower than the subject's body temperature. In certain of these embodiments, the cilnidipine or a pharmaceutically acceptable salt thereof increases the temperature of the vasoconstricted body part to the subject's body temperature at least 5% faster than the non-N-selective calcium channel blocker. For example, the cilnidipine or a pharmaceutically acceptable salt thereof increases the temperature of the vasoconstricted body part to the subject's body temperature at least 10% faster, at least 15% faster, at least 20% faster, at least 25% faster, at least 30% faster, at least 35% faster, at least 40% faster, at least 45% faster, at least 50% faster, at least 55% faster, at least 60% faster, at least 65% faster, at least 70% faster, at least 75% faster, at least 80% faster, at least 85% faster, at least 90% faster, or at least 95% faster than the non-N-selective calcium channel blocker. In some embodiments, the difference can be measured in seconds or minutes.

In the present invention, the disease or disorder is Raynaud's syndrome (e.g., primary Raynaud's syndrome or secondary Raynaud's syndrome); For example, the Raynaud's syndrome is selected from the group consisting of: primary Raynaud's syndrome; secondary Raynaud's syndrome; Raynaud's syndrome of the nipple, nose, ear, penis, tongue, and/or any alar circulatory region. For example, the Raynaud's syndrome is secondary Raynaud's syndrome.

In some embodiments, the subject is also diagnosed with hypertension; and wherein after administration of the cilnidipine or a pharmaceutically acceptable salt thereof to the subject, the blood pressure (e.g., the systolic blood pressure) of the subject is reduced.

In some embodiments, the subject also has hypertension; and wherein after administration of the cilnidipine or a pharmaceutically acceptable salt thereof to the subject, the blood pressure (e.g., the systolic blood pressure) of the subject is reduced. In some embodiments, the subject does not have hypertension; and wherein after administration of the cilnidipine or a pharmaceutically acceptable salt thereof to the subject, the blood pressure (e.g., the systolic blood pressure) of the subject is not reduced. Without wishing to be bound by theory, it is believed that when the subject has hypertension, the dual N-type and L-type calcium channel blocker selective for the N-type calcium channel reduces the blood pressure of the subject; however, when the subject does not have hypertension (i.e., the subject is normotensive), the dual N-type and L-type calcium channel blocker selective for the N-type calcium channel does not reduce the blood pressure of the subject. In some embodiments, after administration of the cilnidipine or a pharmaceutically acceptable salt thereof to the subject, the cardiac function of the subject is improved (e.g., left ventricular function of the subject is improved).

In some embodiments, after administration of the cilnidipine or a pharmaceutically acceptable salt thereof to the subject, the cardiac function of the subject is improved. In some embodiments, improving the cardiac function in the subject comprises improving the left ventricular function of the subject. In some embodiments, the subject has hypertension. In some embodiments, the subject does not have hypertension.

In some embodiments, the subject has hypertension; and after administration of the cilnidipine or a pharmaceutically acceptable salt thereof to the subject, the blood pressure of the subject is decreased and the cardiac function (e.g., left ventricular function) of the subject is improved.

In some embodiments, the subject does not have hypertension; and after administration of the cilnidipine or a pharmaceutically acceptable salt thereof to the subject, the blood pressure of the subject is not decreased and the cardiac function (e.g., left ventricular function) of the subject is improved.

In some embodiments, the subject has hypertension and osteoporosis; and after administration of the cilnidipine or a pharmaceutically acceptable salt thereof to the subject, the blood pressure of the subject is decreased and the bone density in the subject is increased.

In some embodiments, the subject does not have hypertension; the subject has osteoporosis; and after administration of the cilnidipine or a pharmaceutically acceptable salt thereof to the subject, the blood pressure of the subject is not decreased and the bone density in the subject is increased.

In some embodiments, the subject has hypertension; and after administration of the cilnidipine or a pharmaceutically acceptable salt thereof to the subject, the blood pressure of the subject is decreased and the bone density in the subject is increased.

In some embodiments, the subject does not have hypertension; and after administration of the cilnidipine or a pharmaceutically acceptable salt thereof to the subject, the blood pressure of the subject is not decreased and the bone density in the subject is increased.

In some embodiments, the subject has hypertension and atherosclerosis; and after administration of the cilnidipine or a pharmaceutically acceptable salt thereof to the subject, the blood pressure of the subject is decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject does not have hypertension; the subject has atherosclerosis; and after administration of the cilnidipine or a pharmaceutically acceptable salt thereof to the subject, the blood pressure of the subject is not decreased and the atherosclerosis in the subject is improved. In some embodiments, the subject exhibits a reduced amount of plaque deposition in a carotid artery. In some embodiments, the reduced plaque deposition is measured by ultrasound or magnetic resonance imaging.

In some embodiments, the subject has hypertension; and after administration of the cilnidipine or a pharmaceutically acceptable salt thereof to the subject, the blood pressure of the subject is decreased and renal function in the subject is improved.

In some embodiments, the subject does not have hypertension; and after administration of the cilnidipine or a pharmaceutically acceptable salt thereof to the subject, the blood pressure of the subject is not decreased and renal function in the subject is improved.

In some embodiments, the subject has hypertension; and after administration of the cilnidipine or a pharmaceutically acceptable salt thereof to the subject, the blood pressure of the subject is decreased and renal function in the subject is improved. In some embodiments, the subject does not have hypertension; and after administration of the cilnidipine or a pharmaceutically acceptable salt thereof to the subject, the blood pressure of the subject is not decreased and renal function in the subject is improved.

In some embodiments, the subject has hypertension; the subject was previously treated with antihypertensive agents before administration of the cilnidipine or a pharmaceutically acceptable salt thereof; and after administration of the cilnidipine or a pharmaceutically acceptable salt thereof to the subject, the blood pressure of the subject is decreased.

In some embodiments, the subject does not have hypertension; the subject was previously treated with antihypertensive agents before administration of the cilnidipine or a pharmaceutically acceptable salt thereof; and after administration of the cilnidipine or a pharmaceutically acceptable salt thereof to the subject, the blood pressure of the subject is is not decreased.

In some embodiments, the subject has hypertension; the subject has scleroderma; the subject has a digital ulcer; and after administration of the cilnidipine or a pharmaceutically acceptable salt thereof to the subject, the blood pressure of the subject is decreased, and the digital ulcer is treated. In some embodiments, treating the digital ulcer comprises healing or improving the condition of the digital ulcer.

In some embodiments, the subject does not have hypertension; the subject has scleroderma; the subject has a digital ulcer; and after administration of the cilnidipine or a pharmaceutically acceptable salt thereof to the subject, the blood pressure of the subject is not reduced, and the digital ulcer is treated. In some embodiments, treating the digital ulcer comprises healing or improving the condition of the digital ulcer.

In some embodiments, the subject has hypertension; the subject has scleroderma; the disease or disorder associated with dysregulation of blood flow and sympathetic nervous system overactivity is Raynaud's syndrome; and after administration of the cilnidipine or a pharmaceutically acceptable salt thereof to the subject, the blood pressure of the subject is decreased.

In some embodiments, the subject does not have hypertension; the subject has scleroderma; the disease or disorder associated with dysregulation of blood flow and sympathetic nervous system overactivity is Raynaud's syndrome; and after administration of the cilnidipine or a pharmaceutically acceptable salt thereof to the subject, the blood pressure of the subject is not reduced.

In some embodiments, the blood pressure (e.g., systolic blood pressure) of the subject is reduced by greater than 1 mm Hg. For example, the systolic blood pressure of the subject is reduced by greater than 2, 5, 8, 10, 12, 15, 20, 25, or 30 mm Hg. For example, the systolic blood pressure of the subject is reduced by about 1-5 mm Hg, about 5-10 mm Hg, about 10-15 mm Hg, about 15-20 mm Hg, or about 20-30 mm Hg.

In some embodiments, the subject is also being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject has lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject is diagnosed with lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the treatment for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof comprises administering a therapeutic agent. Therapeutic agents known in the art for treating lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof can be found in, e.g., the Physicians' desk reference. (71st ed.). (2017). Montvale, NJ: PDR Network.

In certain embodiments, the subject is being treated for scleroderma. For example, the subject is being treated for scleroderma with interstitial lung disease. In some embodiments, the subject has lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the subject is diagnosed with lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the cilnidipine or a pharmaceutically acceptable salt thereof is administered with an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof. For example, the cilnidipine or a pharmaceutically acceptable salt thereof is administered with nintedanib. In some embodiments, the cilnidipine or a pharmaceutically acceptable salt thereof is administered with a calcineurin inhibitor, a non-steroidal anti-inflammatory drug, or both. In some embodiments, the cilnidipine or a pharmaceutically acceptable salt thereof, the calcineurin inhibitor, and the non-steroidal anti-inflammatory drug are administered separately, sequentially, or simultaneously. In some embodiments, the cilnidipine or a pharmaceutically acceptable salt thereof, the calcineurin inhibitor, and the non-steroidal anti-inflammatory drug are administered separately. In some embodiments, the cilnidipine or a pharmaceutically acceptable salt thereof, the calcineurin inhibitor, and the non-steroidal anti-inflammatory drug are administered sequentially. In some embodiments, the cilnidipine or a pharmaceutically acceptable salt thereof, the calcineurin inhibitor, and the non-steroidal anti-inflammatory drug are administered simultaneously. In some embodiments, the cilnidipine or a pharmaceutically acceptable salt thereof, the calcineurin inhibitor, and the non-steroidal anti-inflammatory drug are administered simultaneously as a fixed dosage form. In some embodiments, the calcineurin inhibitor is a cyclosporine. In some embodiments, the non steroidal anti-inflammatory drug is aspirin.

In certain embodiments, the subject is being treated for lupus. In certain embodiments, the subject is diagnosed with lupus. In certain embodiments, the subject has lupus. In some embodiments, the cilnidipine or a pharmaceutically acceptable salt thereof is administered with an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In certain embodiments, the subject is being treated for rheumatoid arthritis. In certain embodiments, the subject has rheumatoid arthritis. In certain embodiments, the subject is diagnosed with rheumatoid arthritis. In some embodiments, the cilnidipine or a pharmaceutically acceptable salt thereof is administered with an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In certain embodiments, the subject is being treated for Sjögren's syndrome. In certain embodiments, the subject has Sjögren's syndrome. In certain embodiments, the subject is diagnosed with Sjögren's syndrome. In some embodiments, the cilnidipine or a pharmaceutically acceptable salt thereof is administered with an agent selected from the group consisting of: plaquenil, an antimalarial drug (e.g., hydroxychloroquine), evoxac, cevimeline, infliximab, or any combination thereof.

In certain embodiments, the subject is being treated for idiopathic pulmonary fibrosis. In certain embodiments, the subject has idiopathic pulmonary fibrosis. In certain embodiments, the subject is diagnosed with idiopathic pulmonary fibrosis. In some embodiments, the cilnidipine or a pharmaceutically acceptable salt thereof is administered withan agent selected from the group consisting of: nintedanib, pirfenidone, or any combination thereof.

In some embodiments, the disease or disorder characterized by vasoconstriction or neuropathic pain in a subject is secondary Raynaud's disease; the subject is also being treated for scleroderma; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder characterized by vasoconstriction or neuropathic pain in a subject is secondary Raynaud's disease; the subject is also being treated for scleroderma with interstitial lung disease; the dual N-type and L-type calcium channel blocker selective for the N-type calcium channel is cilnidipine; and the subject is further administered an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof (e.g., nintedanib).

In some embodiments, the disease or disorder characterized by vasoconstriction or neuropathic pain in a subject is secondary Raynaud's disease; the subject is also being treated for lupus; the dual N-type and L-type calcium channel blocker selective for the N-type calcium channel is cilnidipine; and the subject is further administered an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In some embodiments, the disease or disorder characterized by vasoconstriction or neuropathic pain in a subject is secondary Raynaud's disease; the subject is also being treated for rheumatoid arthritis; the dual N-type and L-type calcium channel blocker selective for the N-type calcium channel is cilnidipine; and the subject is further administered an agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In some embodiments, the disease or disorder characterized by vasoconstriction or neuropathic pain in a subject is secondary Raynaud's disease; the subject is also being treated for Sjögren's syndrome; the dual N-type and L-type calcium channel blocker selective for the N-type calcium channel is cilnidipine; and the subject is further administered an agent selected from the group consisting of: plaquenil, an antimalarial drug (e.g., hydroxychloroquine), evoxac, cevimeline, infliximab, or any combination thereof.

In some embodiments, the disease or disorder characterized by vasoconstriction or neuropathic pain in a subject is secondary Raynaud's disease; the subject is also being treated for idiopathic pulmonary fibrosis; the dual N-type and L-type calcium channel blocker selective for the N-type calcium channel is cilnidipine; and the subject is further administered an agent selected from the group consisting of: nintedanib, pirfenidone, or any combination thereof.

In some embodiments, the cilnidipine or a pharmaceutically acceptable salt thereof is administered with at least one additional therapeutic agent to the subject. The at least one additional therapeutic agent can be administered simultaneously, separately, sequentially, or in combination (e.g., for more than two agents) with the cilnidipine or a pharmaceutically acceptable salt thereof. Non-limiting examples of additional therapeutic agents include calcium channel blockers, sodium channel blockers (e.g., Nav 1.7 sodium channel blocker), and therapeutic agents that relieve pain.

In certain embodiments, the at least one additional therapeutic agent is selected from the group consisting of: riociguat, amlodipine, nifedipine, nicardipine, conotoxins, cadmium, caroverine, gabapentin, levetiracetam, lamotrigine, NP078585, pregabalin, TROX-1, acetaminophen, non-steroidal anti-inflammatory agents (e.g., ibuprofen), and ziconotide.

In certain embodiments, the at least one additional therapeutic agent is selected from the group consisting of: oxycodone, tramadol, Dilaudid, OxyContin, Cymbalta, a statin, gabapentin, pregabalin, an angiotensin-converting-enzyme (ACE) inhibitor, an angiotensin receptor blocker (ARB), niacin, a proton pump inhibitor, aspirin, Fentanyl Transdermal System, acetaminophen/oxycodone, Roxicodone, Ultram, hydromorphone, Percocet, MS Contin, Butrans, morphine, hydromorphone, methadone, buprenorphine, duloxetine, fentanyl, Duragesic, Endocet, Roxanol, Kadian, Roxicet, ConZip, Methadose, Oxyfast, Dazidox, Fentora, Irenka, Methadone Diskets, Oramorph SR, Roxicodone Intensol, Xtampza ER, Actiq, Belbuca, ETH-Oxydose, Infumorph, naloxone / pentazocine, Oxaydo, Oxydose, OxyIR, ziconotide, Abstral, Astramorph PF, Buprenex, Dolophine, Duramorph, Duramorph PF, Embeda, Lazanda, MorphaBond ER, morphine/naltrexone, Prialt, RMS, Roxanol-T, Sublimaze, Subsys, Talwin Nx, Magnacet, Nalocet, Narvox, Perloxx, Primlev, Xolox, and Prolate.

In another aspect, disclosed herein a compound which is cilnidipine or a pharmaceutically acceptable salt thereof for use in treating secondary Raynaud's syndrome in a subject in need thereof, further comprising administering to the subject at least one additional therapeutic agent.

Without wishing to be bound by theory, it is believed that the secondary Raynaud's syndrome is a consequence of another disease or disorder (also referred to herein as a "primary" disease or disorder). In some embodiments, the primary disease or disorder is lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof. In some embodiments, the at least one additional therapeutic agent is an accepted treatment for the primary disease or disorder.

In certain embodiments, the primary disorder is scleroderma. For example, the primary disorder is scleroderma with interstitial lung disease. In some of the embodiments, the cilnidipine or a pharmaceutically acceptable salt thereof is administered with an agent selected from the group consisting of: a calcineurin inhibitor, cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof. For example, the cilnidipine or a pharmaceutically acceptable salt thereof is administered with nintedanib. In some embodiments, the cilnidipine or a pharmaceutically acceptable salt thereof is administered with a calcineurin inhibitor, a non-steroidal anti-inflammatory drug, or both. In some embodiments, the cilnidipine or a pharmaceutically acceptable salt thereof, the calcineurin inhibitor, and the non-steroidal anti-inflammatory drug are administered separately, sequentially, or simultaneously. In some embodiments, the cilnidipine or a pharmaceutically acceptable salt thereof, the calcineurin inhibitor, and the non-steroidal anti-inflammatory drug are administered separately. In some embodiments, the cilnidipine or a pharmaceutically acceptable salt thereof, the calcineurin inhibitor, and the non-steroidal anti-inflammatory drug are administered sequentially. In some embodiments, the cilnidipine or a pharmaceutically acceptable salt thereof, the calcineurin inhibitor, and the non-steroidal anti-inflammatory drug are administered simultaneously. In some embodiments, the cilnidipine or a pharmaceutically acceptable salt thereof, the calcineurin inhibitor, and the non-steroidal anti-inflammatory drug are administered simultaneously as a fixed dosage form. In some embodiments, the calcineurin inhibitor is a cyclosporine. In some embodiments, the non steroidal anti-inflammatory drug is aspirin.

In certain embodiments, the primary disorder is lupus. In some embodiments, the cilnidipine or a pharmaceutically acceptable salt thereof is administered with an agent selected from the group consisting of: an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof.

In certain embodiments, the primary disorder is rheumatoid arthritis. In some embodiments, the cilnidipine or a pharmaceutically acceptable salt thereof is administered withan agent selected from the group consisting of: disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof.

In certain embodiments, the primary disorder is Sjögren's syndrome. In some embodiments, the cilnidipine or a pharmaceutically acceptable salt thereof is administered with an agent selected from the group consisting of: plaquenil, an antimalarial drug (e.g., hydroxychloroquine), evoxac, cevimeline, infliximab, or any combination thereof.

In the present invention, the dual N-type and L-type calcium channel blocker selective for the N-type calcium channel is cilnidipine or a pharmaceutically acceptable salt thereof.

In some embodiments, the dosage of the cilnidipine or a pharmaceutically acceptable salt thereof is about 0.003 mg/kg to about 5 mg/kg. For example, the dosage of the cilnidipine or a pharmaceutically acceptable salt thereof is about 0.005 mg/kg to about 2 mg/kg, about 0.01 mg/kg to about 1 mg/kg, 0.03 mg/kg to about 0.9 mg/kg, 0.06 mg/kg to about 0.3 mg/kg, about 0.1 mg/kg to about 0.18 mg/kg, or about 1 mg/kg to about 2 mg/kg. In some embodiments, the dosage of the cilnidipine or a pharmaceutically acceptable salt thereof is from about 5 mg to about 50 mg, for example, from about 5 mg to about 15 mg, from about 15 mg to about 25 mg, from about 5 mg to about 25 mg, about 10 mg, or about 20 mg.

Without wishing to be bound by theory, it is believed that dual N-type and L-type calcium channel blocker selective for the N-type calcium channels may decrease the blood pressure (e.g., the systolic blood pressure) of subjects that are hypertensive. As such, it may be beneficial to administer an agent that increases blood pressure (e.g., the systolic blood pressure) in combination with the cilnidipine or a pharmaceutically acceptable salt thereof. In some embodiments, the cilnidipine or a pharmaceutically acceptable salt thereof is administered with a therapeutically effective amount of an agent that increases blood pressure (e.g., the systolic blood pressure). In certain embodiments, the agent that increases blood pressure (e.g., the systolic blood pressure) is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines. In certain embodiments, the blood pressure (e.g., the systolic blood pressure) of the subject before and after administration of the cilnidipine or a pharmaceutically acceptable salt thereof and the agent that increases blood pressure (e.g., the systolic blood pressure) is substantially the same.

In some embodiments, the cilnidipine or a pharmaceutically acceptable salt thereof is administered orally, parenterally, transdermally, by inhalation, intranasally, sublingually, neuraxially, or ocularly

In some embodiments, the bone density of the subject does not decrease. In some of these embodiments, the bone density of the subject increases. This may occur through a reduction in the number of osteoclasts in the subject and/or an increase in the ratio of alkaline phosphate to tartrate resistant acid phosphatase (TRAP).

In some embodiments, the subject is identified or diagnosed as having reduced bone density. In certain embodiments, the subject identified or diagnosed as having reduced bone density is afflicted with osteoporosis. In certain of these embodiments, the subject is female. In some embodiments, after administration of the cilnidipine or a pharmaceutically acceptable salt thereof to the subject, bone density in the subject is increased. Without wishing to be bound by theory, the increase in bone density occurs by means of bone resorption. In some embodiments, after administration of the dual N-type and L-type calcium channel blocker selective for the N-type calcium channel to the subject, osteoporosis in the subject is improved.

In some embodiments, the subject is identified or diagnosed as having reduced renal function. In certain embodiments, the renal function of the subject is not reduced after treatment. In certain embodiments, the renal function of the subject is improved after treatment.

In some embodiments, each administration of the cilnidipine or a pharmaceutically acceptable salt thereof is separated by at least about 12 hours. For example, each administration of the cilnidipine or a pharmaceutically acceptable salt thereof is separated by at least about 24 hours, at least about 30 hours, at least about 48 hours, at least about 60 hours, at least about 72 hours, at least about 4 days, at least about 5 days, at least about 6 days, at least about 1 week, at least about 9 days, at least about 12 days, or at least about 2 weeks.

In some embodiments, the cilnidipine or a pharmaceutically acceptable salt thereof causes sympathetic tone diminution, direct smooth muscle relaxation, dysesthetic pain, burning pain, body temperature changes of the subject, hyperesthesia, changes in skin or tissue color, edema, changes in skin turgor, ruble, pallor, cyanosis, vasospasm, or any combination thereofin the subject.

In some embodiments, the cilnidipine or a pharmaceutically acceptable salt thereof exhibits at least a 5-fold selectivity for the N-type calcium channel over an L-type calcium channel. For example, the cilnidipine or a pharmaceutically acceptable salt thereof exhibits at least a 10-fold, at least a 30-fold, at least a 50-fold, at least a 80-fold, at least a 100-fold, at least a 300-fold, at least a 500-fold, at least a 800-fold, at least a 900-fold, or at least a 1000-fold selectivity for the N-type calcium channel over an L-type calcium channel. For example, the cilnidipine or a pharmaceutically acceptable salt thereof exhibits at least a 50-fold to 100-fold selectivity for the N-type calcium channel over an L-type calcium channel.

In the present invention, the dual N-type and L-type calcium channel blocker selective for the N-type calcium channel further inhibits a sodium channel. The sodium channel is a Nav 1.7 sodium channel. In the present invention, the dual N-type and L-type calcium channel blocker selective for the N-type calcium channel that further inhibits a sodium channel is cilnidipine. In some embodiments, the cilnidipine or pharmaceutically acceptable salt thereof that further inhibits a Nav 1.7 sodium channel is more effective at treating the disease or disorder associated with dysregulation of blood flow and sympathetic nervous system overactivity than an equivalent dose of a non-N-selective calcium channel blocker useful to treat the disease or disorder. In some embodiments, the cilnidipine or pharmaceutically acceptable salt thereof that further inhibits a Nav 1.7 sodium channel is more effective at treating the disease or disorder characterized by neuropathic pain or vasoconstriction than an equivalent dose of a non-N-selective calcium channel blocker useful to treat the disease or disorder.

In some embodiments, after administration of the cilnidipine or pharmaceutically acceptable salt thereof endothelial dysfunction in the subject is improved.

In some embodiments, after administration of the cilnidipine or pharmaceutically acceptable salt thereof endothelial dysfunction in the subject is improved.

In some embodiments, after administration of the cilnidipine or pharmaceutically acceptable salt thereof oxidative stress in the subject is decreased.

In some embodiments, after administration of the cilnidipine or pharmaceutically acceptable salt thereof oxidative stress in the subject is decreased.

In some embodiments, an antioxidant is not administered to the subject. In some embodiments, the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.
In some embodiments, the occurrence of atrial fibrillation is decreased in the subject. Without wishing to be bound by theory, it is believed that decreasing the occurrence of atrial fibrillation in the subject occurs by means of a decrease in autonomic dysfunction.

In some embodiments, the subject is not infected with SARS-CoV-2. In some embodiments, the subject is not being treated for SARS-CoV-2. In some embodiments, the subject is not diagnosed with SARS-CoV-2 (e.g., after the subject is subjected to a test that diagnoses whether the subject is infected with SARS-CoV-2 (e.g., a PCR test, an antigen test, or an antibody test)).

### Pharmaceutical Compositions and Formulations

Disclosed herein is a pharmaceutical composition comprising cilnidipine or a pharmaceutically acceptable salt thereof, an agent that increases blood pressure, and optionally a pharmaceutically acceptable excipient.

In another aspect, disclosed herein is a pharmaceutical composition comprising cilnidipine or a pharmaceutically acceptable salt thereof; a therapeutic agent selected from the group consisting of: oxycodone, tramadol, Dilaudid, OxyContin, Cymbalta, a statin, gabapentin, pregabalin, an angiotensin-converting-enzyme (ACE) inhibitor, an angiotensin receptor blocker (ARB), niacin, a proton pump inhibitor, aspirin, Fentanyl Transdermal System, acetaminophen/oxycodone, Roxicodone, Ultram, hydromorphone, Percocet, MS Contin, Butrans, morphine, hydromorphone, methadone, buprenorphine, duloxetine, fentanyl, Duragesic, Endocet, Roxanol, Kadian, Roxicet, ConZip, Methadose, Oxyfast, Dazidox, Fentora, Irenka, Methadone Diskets, Oramorph SR, Roxicodone Intensol, Xtampza ER, Actiq, Belbuca, ETH-Oxydose, Infumorph, naloxone / pentazocine, Oxaydo, Oxydose, OxyIR, ziconotide, Abstral, Astramorph PF, Buprenex, Dolophine, Duramorph, Duramorph PF, Embeda, Lazanda, MorphaBond ER, morphine/naltrexone, Prialt, RMS, Roxanol-T, Sublimaze, Subsys, Talwin Nx, Magnacet, Nalocet, Narvox, Perloxx, Primlev, Xolox, and Prolate; and optionally a pharmaceutically acceptable excipient.

In some embodiments, the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.

Disclosed herein is a pharmaceutical composition comprising cilnidipine or a pharmaceutically acceptable salt thereof, an agent that treats erectile dysfunction, and optionally a pharmaceutically acceptable excipient.

In some embodiments, the agent that treats erectile dysfunction is sildenafil, tadafenil, or phosphodiesterase type 5 inhibitors.

Disclosed herein is a pharmaceutical composition comprising cilnidipine or a pharmaceutically acceptable salt thereof; cyclophosphamide, nintedanib, methotrexate, mycophenolate, a glucocorticoid (e.g., prednisone, dexamethasone, and hydrocortisone), a non steroidal anti-inflammatory drug (e.g., aspirin, ibuprofen, or naproxen), D-penicillamine, a diuretic, omeprazole, bosentan, epoprostenol, enalapril, Lisinopril, captopril, or any combination thereof; and optionally a pharmaceutically acceptable excipient.

Disclosed herein is a pharmaceutical composition comprising cilnidipine or a pharmaceutically acceptable salt thereof; nintedanib; and optionally a pharmaceutically acceptable excipient.

Disclosed herein is a pharmaceutical composition comprising cilnidipine or a pharmaceutically acceptable salt thereof; an antimalarial drug (e.g., hydroxychloroquine), a non-steroidal anti-inflammatory drug (e.g., aspirin, ibuprofen, or naproxen), belimumab, a corticosteroid (e.g., prednisone or prednisolone), an immunosuppressant (e.g., azathioprine, cyclophosphamide, methotrexate, and mycophenolate mofetil), or any combination thereof; and optionally a pharmaceutically acceptable excipient.

Disclosed herein is a pharmaceutical composition comprising cilnidipine or a pharmaceutically acceptable salt thereof; disease-modifying anti-rheumatic drugs (e.g., methotrexate or sulfasalazine), a non-steroidal anti-inflammatory drug (e.g., aspirin, ibuprofen, or naproxen), a corticosteroid (e.g., prednisone or prednisolone), a biologic (e.g., anakinra or tocilizumab), or any combination thereof; and optionally a pharmaceutically acceptable excipient.

Disclosed herein is a pharmaceutical composition comprising cilnidipine or a pharmaceutically acceptable salt thereof; plaquenil, an antimalarial drug (e.g., hydroxychloroquine), evoxac, cevimeline, infliximab, or any combination thereof; and optionally a pharmaceutically acceptable excipient.

Disclosed herein is a pharmaceutical composition comprising cilnidipine or a pharmaceutically acceptable salt thereof; nintedanib, pirfenidone, or any combination thereof; and optionally a pharmaceutically acceptable excipient.

Disclosed herein is a pharmaceutical composition comprising cilnidipine or a pharmaceutically acceptable salt thereof, a calcineurin inhibitor, and optionally a pharmaceutically acceptable excipient.

In some embodiments, the calcineurin inhibitor is a cyclosporine.

In some embodiments, the pharmaceutical composition further comprises a non-steroidal anti-inflammatory drug. In some embodiments, the non steroidal anti-inflammatory drug is aspirin.

In some embodiments, a chemical entity (e.g., the cilnidipine or a pharmaceutically acceptable salt thereof)) is administered as a pharmaceutical composition that includes the cilnidipine or a pharmaceutically acceptable salt thereof and one or more pharmaceutically acceptable excipients, and optionally one or more additional therapeutic agents as described herein.

In some embodiments, the cilnidipine or a pharmaceutically acceptable salt thereof can be administered in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkylcyclodextrins, including 2- and 3-hydroxypropyl-β-cyclodextrins, or other solubilized derivatives can also be used to enhance delivery of compounds described herein. Dosage forms or compositions containing a chemical entity as described herein in the range of 0.005% to 100% with the balance made up from non-toxic excipient may be prepared. The contemplated compositions may contain 0.001%-100% of a chemical entity provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

### Routes of Administration and Composition Components

In some embodiments, the chemical entities described herein or a pharmaceutical composition thereof can be administered to subject in need thereof by any accepted route of administration. Acceptable routes of administration include, but are not limited to, oral, parenteral, transdermal, intranasal, sublingual, neuraxial, or ocular.

Compositions can be formulated for parenteral administration, e.g., formulated for injection via the intravenous, intramuscular, sub-cutaneous, or even intraperitoneal routes. Typically, such compositions can be prepared as injectables, either as liquid solutions or suspensions; solid forms suitable for use to prepare solutions or suspensions upon the addition of a liquid prior to injection can also be prepared; and the preparations can also be emulsified. The preparation of such formulations will be known to those of skill in the art in light of the present disclosure.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

In other embodiments, the compounds described herein or a pharmaceutical composition thereof are suitable for local delivery to the digestive or GI tract by way of oral administration (e.g., solid or liquid dosage forms.).

Solid dosage forms for oral administration include capsules, tablets, pills, powders, and granules. In such solid dosage forms, the chemical entity is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the compositions will take the form of a unit dosage form such as a pill or tablet and thus the composition may contain, along with a chemical entity provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (e.g., in propylene carbonate, vegetable oils, PEG's, poloxamer 124 or triglycerides) is encapsulated in a capsule (gelatin or cellulose base capsule). Unit dosage forms in which one or more chemical entities provided herein or additional active agents are physically separated are also contemplated; e.g., capsules with granules (or tablets in a capsule) of each drug; two-layer tablets; two-compartment gel caps, etc. Enteric coated or delayed release oral dosage forms are also contemplated.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid.

In certain embodiments the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

In certain embodiments, solid oral dosage forms can further include one or more components that chemically and/or structurally predispose the composition for delivery of the chemical entity to the stomach or the lower GI; e.g., the ascending colon and/or transverse colon and/or distal colon and/or small bowel. Exemplary formulation techniques are described in, e.g., Filipski, K.J., et al., Current Topics in Medicinal Chemistry, 2013, 13, 776-802.

Examples include upper-GI targeting techniques, e.g., Accordion Pill (Intec Pharma), floating capsules, and materials capable of adhering to mucosal walls.

Other examples include lower-GI targeting techniques. For targeting various regions in the intestinal tract, several enteric/pH-responsive coatings and excipients are available. These materials are typically polymers that are designed to dissolve or erode at specific pH ranges, selected based upon the GI region of desired drug release. These materials also function to protect acid labile drugs from gastric fluid or limit exposure in cases where the active ingredient may be irritating to the upper GI (e.g., hydroxypropyl methylcellulose phthalate series, Coateric (polyvinyl acetate phthalate), cellulose acetate phthalate, hydroxypropyl methylcellulose acetate succinate, Eudragit series (methacrylic acid-methyl methacrylate copolymers), and Marcoat). Other techniques include dosage forms that respond to local flora in the GI tract, Pressure-controlled colon delivery capsule, and Pulsincap.

Ocular compositions can include, without limitation, one or more of any of the following: viscogens (e.g., Carboxymethylcellulose, Glycerin, Polyvinylpyrrolidone, Polyethylene glycol); Stabilizers (e.g., Pluronic (triblock copolymers), Cyclodextrins); Preservatives (e.g., Benzalkonium chloride, ETDA, SofZia (boric acid, propylene glycol, sorbitol, and zinc chloride; Alcon Laboratories, Inc.), Purite (stabilized oxychloro complex; Allergan, Inc.)).

Topical compositions can include ointments and creams. Ointments are semisolid preparations that are typically based on petrolatum or other petroleum derivatives. Creams containing the selected active agent are typically viscous liquid or semisolid emulsions, often either oil-in-water or water-in-oil. Cream bases are typically water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. As with other carriers or vehicles, an ointment base should be inert, stable, nonirritating and non-sensitizing.

In any of the foregoing embodiments, pharmaceutical compositions described herein can include one or more one or more of the following: lipids, interbilayer crosslinked multilamellar vesicles, biodegradeable poly(D,L-lactic-co-glycolic acid) [PLGA]-based or poly anhydride-based nanoparticles or microparticles, and nanoporous particle-supported lipid bilayers.

### Regimens

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, each administration of the cilnidipine or a pharmaceutically acceptable salt thereof is separated by at least about 12 hours. For example, each administration of the cilnidipine or a pharmaceutically acceptable salt thereof is separated by at least about 24 hours, at least about 30 hours, at least about 48 hours, at least about 60 hours, at least about 72 hours, at least about 4 days, at least about 5 days, at least about 6 days, at least about 1 week, at least about 9 days, at least about 12 days, or at least about 2 weeks.

In some embodiments, the period of administration of a compound described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In a further embodiment, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 1 1 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

### EXAMPLES

Example 1: Cell based assay activity of cilnidipine, gabapentin, amlodipine, tetrodotoxin (TTX), and bupivacaine.

### Scope

The aim of this assay was to record IC₅₀ values for 4 compounds (defined below) on the voltage-gated sodium channel subtype 1.7 (Nav 1.7) on both the closed and the inactivated state using the automated patch clamp platform QPATCH II^{™}.

### Background

The Nav 1.7 sodium channel is expressed in the dorsal root ganglion (DRG) and plays a role in propagating the action potential in these cells. Opening of the Nav 1.7 sodium channel depolarizes the membrane potential. Following this depolarization, the channel enters an inactivated state that prevents the channel from "firing" again too early. After the cells' membrane potential has returned to the resting potential, the channel is slowly released from the inactivated state into the closed (resting) state. A state dependent block of a sodium channel.

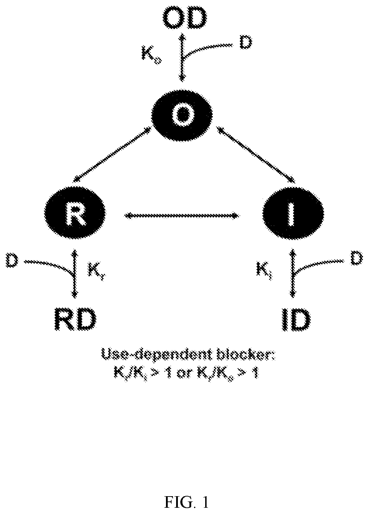

FIG. 1 is a schematic diagram based on the modulated hypothesis of drug binding to various states of a sodium channel (NaCh). *See* Benjamin et al. 2006 State-dependent compound inhibition of Nav 1.2 sodium channels using the FLIPR Vm dye: on-target and off-target effects of diverse pharmacological agents. J Biomol Screen. 2006 Feb; 11(1):29-39*.* The states represented are as follows: R (resting), O (open), I (inactivated). The model shows that a drug can directly interact with any of the 3 states. When a drug exhibits the same affinity for all 3 states, it is considered non-state dependent. When a drug exhibits greater affinity for 1 or 2 states of the channel than it does for the third, it is considered state-dependent. When a drug exhibits greater affinity for the open or inactivated states than it does for the resting state, it is considered use-dependent. A state-dependent blocker will act more potently in cells that fire a high frequency of action potentials. For this reason, drug development against voltage - gated sodium channels typically includes protocols that allow to distinguish between the modes of action.

### Materials and Methods:

### Compounds:

Cilnidipine, gabapentin, and amlodipine were obtained from SIGMA ALDRICH^{®}, tetrodotoxin (TTX) was obtained from ALOMONE LABS^{®}, and bupivacaine was obtained from AUROMEDICS^{®}. All the compounds were added to the external solution and a serial dilution of 6 concentrations were performed for each compound based on clinical Cₘₐₓ values.

### Cell line:

Chinese Hamster Ovary (CHO) cells stably expressing human Nav 1.7 were cultured according to the manufacturer's standard operating procedure (SOP) and enzymatically lifted into solution following Sophion's standard SOP.

### Solutions:

### External Solution: EC 0.0.0 NaCl-Ringer's Solution

| Chemical | Total Concentration |
|---|---|
| CaCl₂ (ID 2) | 2 mM |
| MgCl₂ (ID 15) | 1 mM |
| HEPES | 10 mM |
| KCL (ID 12) | 4 mM |
| NaCl (ID 17) | 145 mM |
| Glucose (ID 8) | 10 mM |

### Internal Solution: IC 7.0.0 KF-Ringer

| Chemical | Total Concentration |
|---|---|
| KF | 120 mM |
| KCL (ID 12) | 20 mM |
| HEPES | 10 mM |
| EGTA | 10 mM |
| pH: 7.2 mOsm with KOH: 300 mOsm | |

### Reference:

### 3 µM TTX in external solution

### Voltage Protocols:

Voltage protocol 1 (VP1): A voltage protocol designed to generate a current voltage relationship and to estimate the half inactivation potential (V1/2) was used. The first voltage protocol was used as quality control and the latter was used later in the experiment (see below).

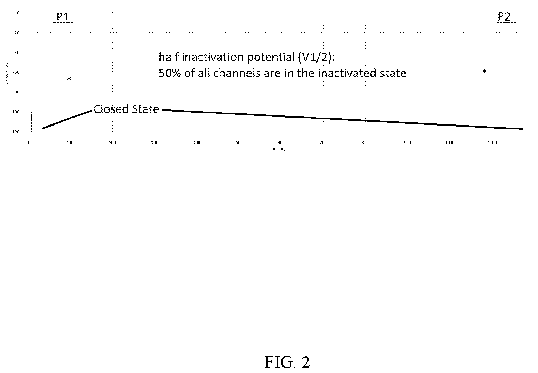

Voltage protocol 2 (VP2): Two pulse voltage protocol. FIG. 2 depicts a two pulse voltage protocol. The line marked with an asterisk indicates the voltage that was applied to the cell. In the beginning, all channels were in the closed state (V = -120 mV). A depolarization step to V = 10 mV (P1) activated all channels and produced the largest possible current. Next, cells were kept at the V1/2 value for 1 s. Compounds that have an affinity for this state had the possibility to bind to this state during this time. A second stimulation to V = -10 mV activated all channels that were in the closed state (50%). Compound effects were assessed at both P1 and P2. State dependent compounds will show a different degree of inhibition at the two different pulses. This protocol was repeated every 10 s to monitor compound effects on the channel.

### Application Protocol:

### Liquid Periods

| Liquid | Volume (µl) | VP runs | Details |
|---|---|---|---|
| 1. Res: Saline | 5 | 12 | N/A |
| 2. Res: Saline | 5 | 1 | N/A |
| 3. Res: Saline | 0 | 6 | N/A |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 4. MTP: concentration 1 | 5 | 18 | 3 repetitions |
| 10 Res: Reference | 5 | 12 | N/A |

### Results:

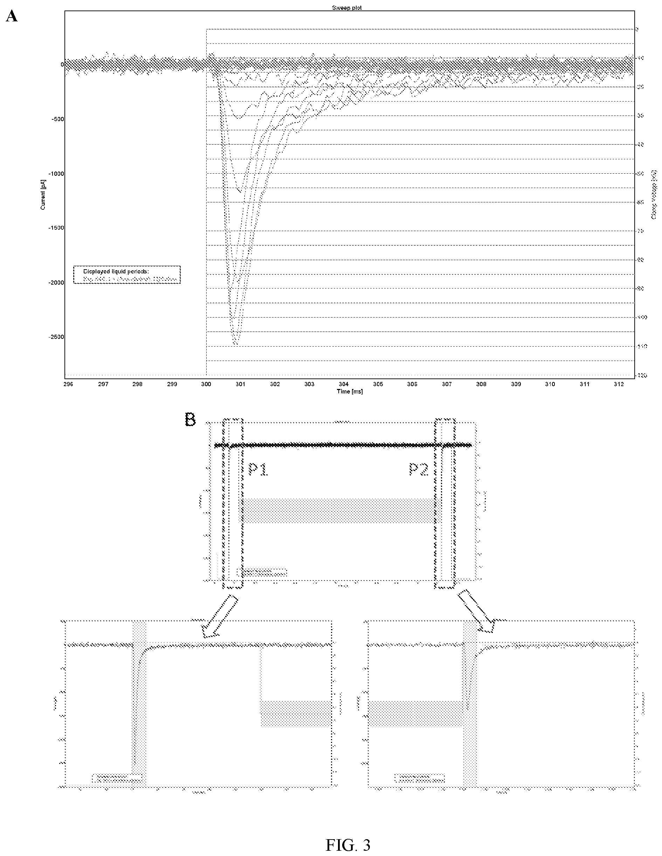

Raw traces can be seen in FIG. 3A and FIG. 3B. FIG. 3A is an overlay of inactivation current traces from VP1. FIG. 3B depicts current traces at closed and inactivated states from the two pulse voltage protocol (VP2). Current traces were expanded to indicate the current difference between the closed (P1) and inactivated (P2) states.

*Current over time plots for all compounds showing the decrease in current following compound exposure.*

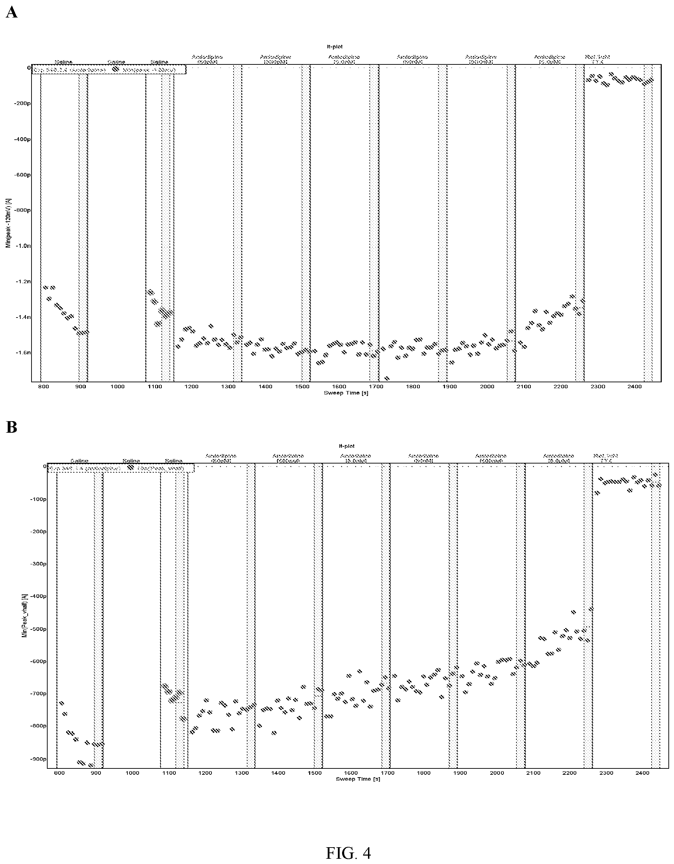

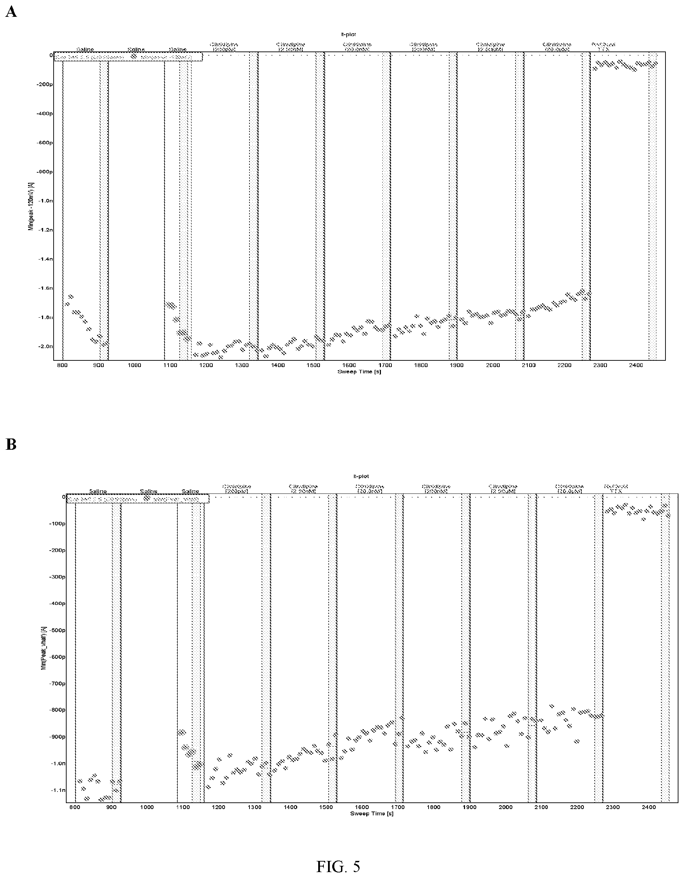

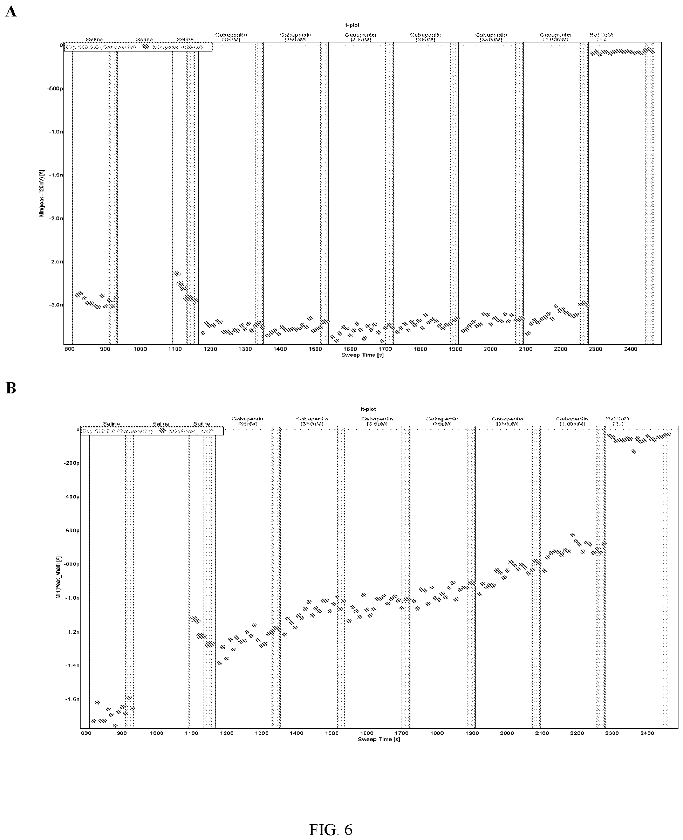

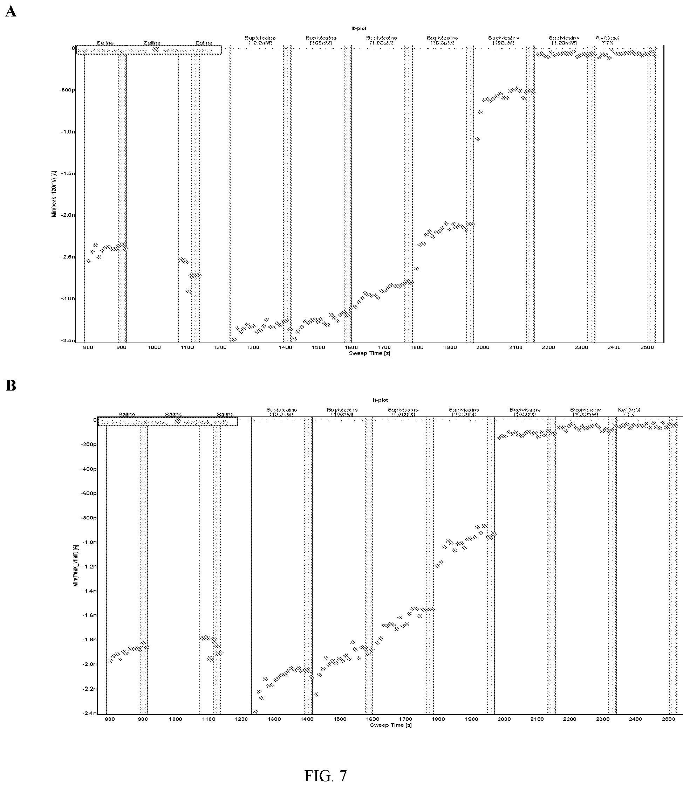

FIG. 4A depicts an It-plot for amlodipine at the closed state (P1). FIG. 4B depicts an It-plot for amlodipine at the inactivated state (P2). Concentrations of amlodipine were: 50pM, 500pM, 5nM, 50nM, 500nM, and 5µM. FIG. 5A depicts an It-plot for cilnidipine at the closed state (P1). FIG. 5B is an It-plot for cilnidipine at the inactivated state (P2). FIG. 6A depicts an It-plot for gabapentin at closed state (P1). FIG. 6B depicts an It-plot for gabapentin at inactivated state (P2). Concentrations of gabapentin were: 35nM, 350nM, 3.5µM, 35µM, 350µM, and 1mM. Concentrations of cilnidipine were: 200pM, 2nM, 20nM, 200nM, 2µM, and 20µM. FIG. 7A depicts an example of an It-plot for bupivacaine at closed state (P1). FIG. 7B depicts an It-plot for bupivacaine at inactivated state (P2). Concentrations of bupivacaine were: 10nM, 100nM, 1µM, 10µM, 100µM, and 1mM.

*Combined concentration response curve for the closed (P1) and the inactivated state (P2) for each compound.*

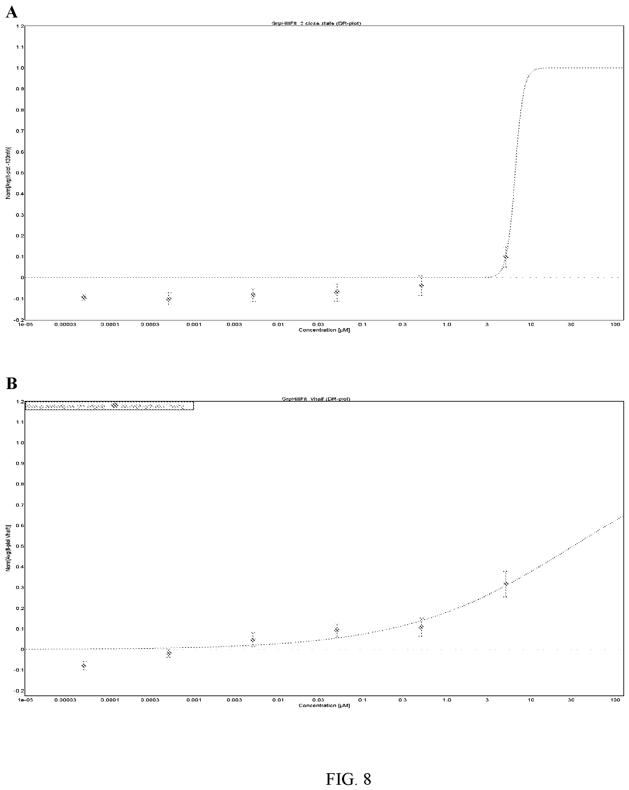

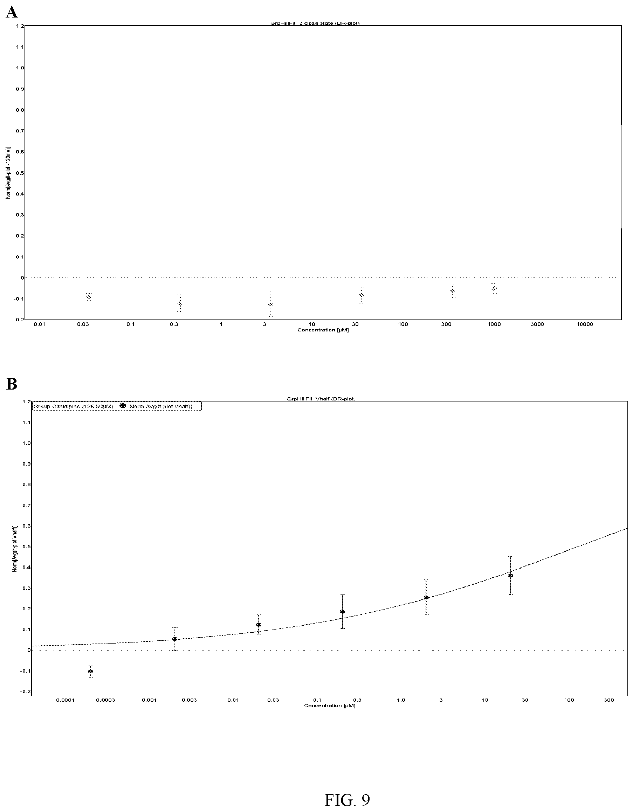

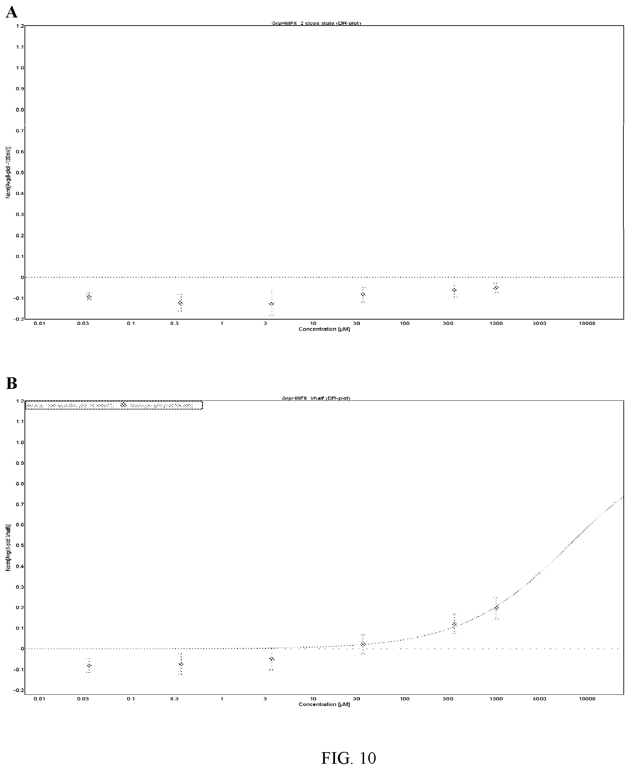

FIG. 8A depicts a group hill fit for amlodipine at closed state (P1) and FIG. 8B depicts a group hill fit for amlodipine at (P2) inactivated state. X-axis is the compound concentration and Y-axis is the inhibited rate with 1 set as maximum. FIG. 9A depicts a group hill fit for cilnidipine at closed state (P1) and FIG. 9B depicts a group hill fit for cilnidipine at (P2) inactivated state. The X-axis is the compound concentration and Y-axis is the inhibited rate with 1 set as maximum. FIG. 10A depicts a group hill fit for gabapentin at closed state (P1) and FIG. 10B depicts (P2) inactivated state. The X-axis is the compound concentration and the Y-axis is the inhibited rate with 1 set as maximum.

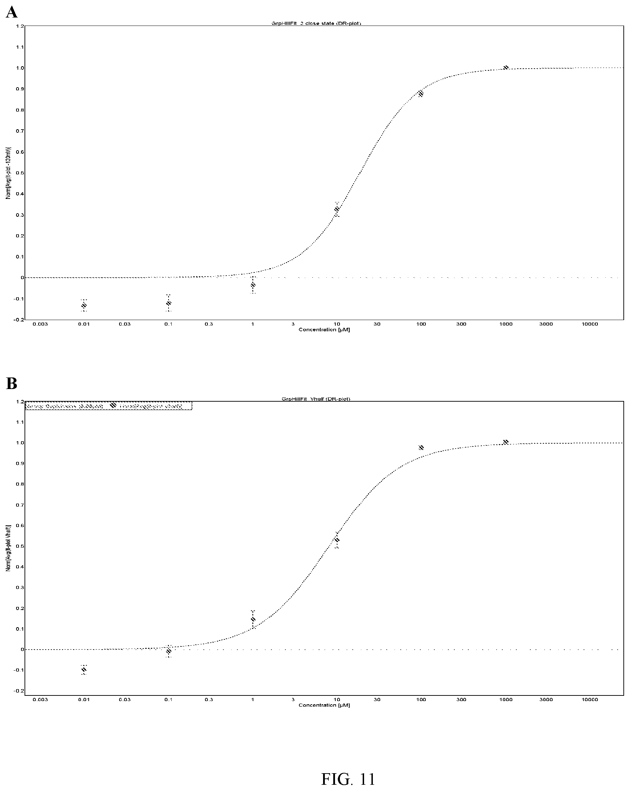

FIG. 11A depicts a group hill fit for bupivacaine at closed state (P1) and FIG. 11B depicts (P2) inactivated state. The X-axis is the compound concentration and the Y-axis is the inhibited rate with 1 set as maximum.

### Summarized relative Nav 1.7 channel inhibition

At the plasma concentrations achieved with a 10 mg dose of cilnidipine, versus a clinically comparable 5 mg dose of amlodipine, cilnidipine has a 9.5% higher relative inhibition of Nav 1.7 than amlodipine.

**Example 2:** Relative Nav 1.7 inhibition of the Nav 1.7 sodium channel of amlodipine, cilnidipine, nifedipine, gabapentin, bupivacaine, lidocaine, and DMSO.

### Materials and Methods:

The voltage protocol for this experiment are the same as described in connection with Example 1.

**Table 4. Testing concentration for each compound.**

| Compound | Oral dose of compound | Cmax^{†} | Cmax used in experiments | Concentrations tested |
|---|---|---|---|---|
| nifedipine | 40 mg | 36.55 ± 6.76 ng/ml | 40mg/ml (~115nM) | 0.1 µM |
| cilnidipine | 10 mg | 5.9 ± 1.2 ng/ml | 10ng/ml (~20nM) | 0.02 µM |
| cilnidipine | 20 mg | 37.0 | N/A | N/A |
| amlodipine | 10 mg | 8.43 ± 2.24 µg/ml | 2ng/ml (~4nM) | 0.005 µM |
| gabapentin | 1800 mg | N/A | 6µg/ml (~35µM) | 35 µM |
| bupvicacaine HCl* | N/A | N/A | N/A | 1 µM |
| lidocaine HCl* | N/A | N/A | N/A | 10 µM |
| DMSO* | N/A | N/A | N/A | N/A |

| | | | | |
|---|---|---|---|---|
| * Denotes reference compound †Denotes reference value | | | | |

### Summary of inhibition rate for closed and inactivated state at/around Cmax concentrations.

**Table 5. Experimental data of mean values of inhibition for closed and inactivated state of the Nav 1.7 sodium channel.**

| Compound | Mean % inhibition-closed state | Mean % inhibition-inactivated state | Std. error closed state | Std. error inhibition state |
|---|---|---|---|---|
| nifedipine | -2.2 | 37.5 | 2.0 | 3.7 |
| cilnidipine | -6.7 | 18.5 | 3.7 | 3.1 |
| amlodipine | -7.4 | 7.8 | 3.8 | 0.2 |
| gabapentin | -5.2 | 6.3 | 3.2 | 0.1 |
| bupivacaine | 2.5 | 20 | 2.4 | 3.6 |
| lidocaine | 40.9 | 89.0 | 3.3 | 2.1 |
| DMSO | -2.7 | 38.7 | 2.9 | 4.9 |

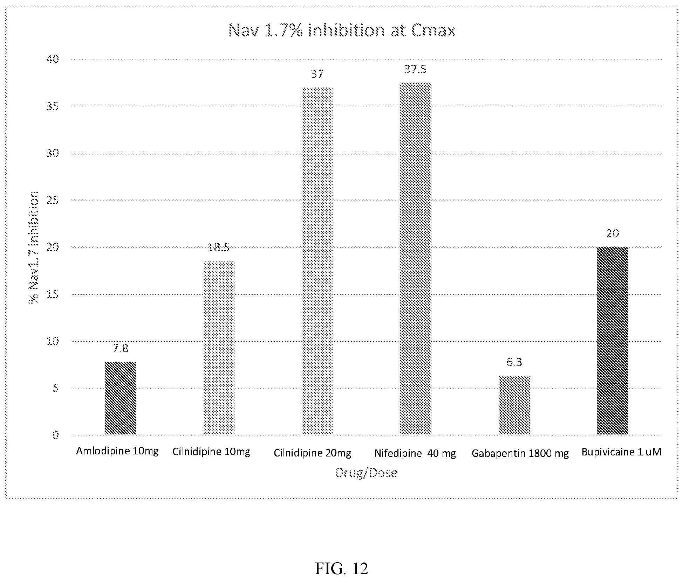

FIG. 12 depicts percent Nav 1.7 inactivated state sodium channel inhibition at Cₘₐₓ for nifedipine 40 mg, cilnidipine 10 mg, cilnidipine 20 mg, amlodipine 10 mg, gabapentin 1800 mg, and bupivacaine 1 µM.

### Results

Cilnidipine has clinically meaningful activity at the sodium channel subunit Nav 1.7, a target in the development of treatments for neuropathic pain. The activity of cilnidipine was about 5X that of amlodipine at comparable clinical doses (amlodipine 5 mg to cilnidipine 10 mg and amlodipine 10 mg to cilnidipine 20 mg). In addition, cilnidipine at 20 mg dose shows activity that is greater than gabapentin, another drug used to treat neuropathic pain. The activity of cilnidipine at a 20 mg dose appears to be greater than a sodium channel blocker and local anesthetic, bupivacaine, which is used sometimes to block postoperative pain with regional anesthesia or wound infiltration. The data shows, that the compounds in terms of efficacy would be ranked gabapentin < amlodipine < cilnidipine -nifedipine.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A compound which is cilnidipine or a pharmaceutically acceptable salt thereof, for use in treating Raynaud's syndrome in a subject in need thereof.

2. The compound for use of claim 1, wherein Raynaud's syndrome is selected from the group consisting of primary Raynaud's syndrome; secondary Raynaud's syndrome; Raynaud's syndrome of the nipple, nose, ear, penis, tongue, and/or any alar circulatory region, optionally wherein the Raynaud's syndrome is secondary Raynaud's syndrome.

3. The compound for use of claims 1 or 2, wherein the subject is also diagnosed with hypertension; and wherein after administration of cilnidipine to the subject, the blood pressure of the subject is reduced, optionally wherein the systolic blood pressure of the subject is reduced by greater than 10 mm Hg.

4. The compound for use of any one of claims 1 to 3, wherein the subject is being treated for lupus, scleroderma, scleroderma with interstitial lung disease, idiopathic pulmonary fibrosis, primary pulmonary hypertension, rheumatoid arthritis, atherosclerosis, cryoglobulinemia, polycythemia, dermatomyositis, polymyositis, Sjögren's syndrome, or any combination thereof, optionally wherein the subject is being treated for scleroderma, optionally wherein the subject is being treated for scleroderma with interstitial lung disease, optionally wherein the subject is being treated for lupus, optionally wherein the subject is being treated for rheumatoid arthritis, optionally wherein the subject is being treated for Sjögren's syndrome, optionally wherein the subject is being treated for idiopathic pulmonary fibrosis.

5. The compound for use of claim 4, further comprising an agent selected from the group consisting of a calcineurin inhibitor, cyclophosphamide, nintedanib, methotrexate, mycophenolate, a glucocorticoid, a non steroidal anti-inflammatory drug, D-penicillamine, a diuretic, omeprazole, bosentan, epoprostenol, enalapril, lisinopril, captopril, or any combination thereof, optionally further comprising nintedanib, optionally further comprising a calcineurin inhibitor, a non-steroidal anti-inflammatory drug, or both, optionally wherein the calcineurin inhibitor is a cyclosporine and optionally, wherein the non steroidal anti-inflammatory drug is aspirin or further comprising an agent selected from the group consisting of an antimalarial drug, a non-steroidal anti-inflammatory drug, belimumab, a corticosteroid, an immunosuppressant, or any combination thereof or further comprising an agent selected from the group consisting of methotrexate, sulfasalazine, a non-steroidal anti-inflammatory drug, a corticosteroid, a biologic, or any combination thereof, or further comprising an agent selected from the group consisting of plaquenil, an antimalarial drug, evoxac, cevimeline, infliximab, or any combination thereof, or further comprising an agent selected from the group consisting of: nintedanib, pirfenidone, or any combination thereof.

6. The compound for the use of any one of claims 1 to 5, comprising at least one additional calcium channel blocker, optionally wherein the at least one additional calcium channel blocker is selected from the group consisting of: amlodipine, nifedipine, nicardipine, nimodipine, verapamil, diltiazem, felodipine, isradipine, nisoldipine, and nitrendipine.

7. The compound for use of any one of claims 1 to 6, further comprising a therapeutically effective amount of an agent that increases blood pressure, optionally wherein the agent that increases blood pressure is selected from the group consisting of: midodrine, cortisone, prednisone, trimipramine, venlafaxine, anabolic steroids, antidepressants, anti-obesity drugs, CETP inhibitors, herbal preparations, immunosuppressants, mineralocorticoids, NSAIDS/coxibs, serotonergics, stimulants, sulfonylureas, and sympathomimetic amines.

8. The compound for use of any one of claim 7, wherein the blood pressure of the subject before and after administration of the cilnidipine and the agent that increases blood pressure is substantially the same.

9. The compound for the use of claims 1 to 8, wherein the bone density of the subject does not decrease.

10. The compound for the use of any one of claims 1 to 9, wherein the subject is identified or diagnosed as having reduced bone density for the treatment, optionally wherein the subject identified or diagnosed as having reduced bone density is afflicted with osteoporosis, optionally wherein the subject is female.

11. The compound for the use of claims 1 to 10, wherein the subject is identified or diagnosed as having reduced renal function for the treatment, optionally wherein the renal function of the patient is not reduced after treatment.

12. The compound for the use of any one of claims 1 to 11, wherein the dosage of cilnidipine is about 0.005 mg/kg to about 2 mg/kg, optionally wherein the dosage is about 0.06 mg/kg to about 0.3 mg/kg, optionally wherein the dosage is about 0.1 mg/kg to about 0.18 mg/kg.

13. The compound for the use of any one of claims 1 to 12, wherein the cilnidipine is administered orally, parenterally, transdermally, by inhalation, intranasally, sublingually, neuraxially, or ocularly.

14. The compound for the use of any one of claims 1 to 13, wherein each administration of the cilnidipine is separated by at least about 24 hours, optionally by at least about 48 hours, optionally by at least about 72 hours, optionally by at least about 1 week.

15. The compound for the use of any one of claims 1 to 14, wherein an antioxidant is not administered to the subject, optionally wherein the anti-oxidant is selected from the group consisting of a hydralazine compound, a glutathione, vitamin C, cysteine, β-carotene, a ubiquinone, a ubiquinol-10, a tocopherol, coenzyme Q, or a mixture thereof.

## Patentansprüche

1. Verbindung, bei der es sich um Cilnidipin oder ein pharmazeutisch unbedenkliches Salz davon handelt, zur Verwendung bei der Behandlung von Raynaud-Syndrom bei einem behandlungsbedürftigen Individuum.

2. Verbindung zur Verwendung nach Anspruch 1, wobei das Raynaud-Syndrom aus der Gruppe bestehend aus primärem Raynaud-Syndrom, sekundärem Raynaud-Syndrom, Raynaud-Syndrom von Brustwarze, Nase, Ohr, Penis, Zunge und/oder einer Alar-Kreislaufregion ausgewählt ist, gegebenenfalls wobei es sich bei dem Raynaud-Syndrom um ein sekundäres Raynaud-Syndrom handelt.

3. Verbindung zur Verwendung nach Anspruch 1 oder 2, wobei bei dem Individuum auch Hypertonie diagnostiziert wurde und wobei nach Verabreichung von Cilnidipin an das Individuum der Blutdruck des Individuums herabgesetzt ist, gegebenenfalls wobei der systolische Blutdruck des Individuums um mehr als 10 mmHg herabgesetzt ist.

4. Verbindung zur Verwendung nach einem der Ansprüche 1 bis 3, **dadurch gekennzeichnet, dass** das Individuum gegen Lupus, Sklerodermie, Sklerodermie mit interstitieller Lungenerkrankung, idiopathische Lungenfibrose, primäre pulmonale Hypertonie, rheumatoide Arthritis, Atherosklerose, Kryoglobulinämie, Polyzythämie, Dermatomyositis, Polymyositis, Sjögren-Syndrom oder eine Kombination davon behandelt wird, gegebenenfalls wobei das Individuum gegen Sklerodermie behandelt wird, gegebenenfalls wobei das Individuum gegen Sklerodermie mit interstitieller Lungenerkrankung behandelt wird, gegebenenfalls wobei das Individuum gegen Lupus behandelt wird, gegebenenfalls wobei das Individuum gegen rheumatoide Arthritis behandelt wird, gegebenenfalls wobei das Individuum gegen Sjögren-Syndrom behandelt wird, gegebenenfalls wobei das Individuum gegen idiopathische Lungenfibrose behandelt wird.

5. Verbindung zur Verwendung nach Anspruch 4, ferner umfassend ein Mittel, das aus der Gruppe bestehend aus einem Calcineurin-Inhibitor, Cyclophosphamid, Nintedanib, Methotrexat, Mycophenolat, einem Glucocorticoid, einem nichtsteroidalen Antiphlogistikum, D-Penicillamin, einem Diuretikum, Omeprazol, Bosentan, Epoprostenol, Enalapril, Lisinopril, Captopril oder einer Kombination davon ausgewählt ist, gegebenenfalls weiter umfassend Nintedanib, gegebenenfalls weiter umfassend einen Calcineurin-Inhibitor, ein nichtsteroidales Antiphlogistikum oder beide, gegebenenfalls wobei es sich bei dem Calcineurin-Inhibitor um ein Cyclosporin handelt und gegebenenfalls, wobei es sich bei dem nichtsteroidalen Antiphlogistikum um Aspirin handelt, oder ferner umfassend ein Mittel, das aus der Gruppe bestehend aus einem Antimalariamittel, einem nichtsteroidalen Antiphlogistikum, Belimumab, einem Corticosteroid, einem Immunsuppressivum oder einer Kombination davon ausgewählt ist, oder ferner umfassend ein Mittel, das aus der Gruppe bestehend aus Methotrexat, Sulfasalazin, einem nichtsteroidalen Antiphlogistikum, einem Corticosteroid, einem Biologikum oder einer Kombination davon ausgewählt ist, oder ferner umfassend ein Mittel, das aus der Gruppe bestehend aus Plaquenil, einem Antimalariamittel, Evoxac, Cevimelin, Infliximab oder einer Kombination davon ausgewählt ist, oder ferner umfassend ein Mittel, das aus der Gruppe bestehend aus Nintedanib, Pirfenidon oder einer Kombination davon ausgewählt ist.

6. Verbindung zur Verwendung nach einem der Ansprüche 1 bis 5, umfassend wenigstens einen zusätzlichen Calciumkanalblocker, gegebenenfalls wobei der wenigstens eine zusätzliche Calciumkanalblocker aus der Gruppe bestehend aus Amlodipin, Nifedipin, Nicardipin, Nimodipin, Verapamil, Diltiazem, Felodipin, Isradipin, Nisoldipin und Nitrendipin ausgewählt ist.

7. Verbindung zur Verwendung nach einem der Ansprüche 1 bis 6, ferner umfassend eine therapeutisch wirksame Menge eines blutdrucksteigernden Mittels, gegebenenfalls wobei das blutdrucksteigernde Mittel aus der Gruppe bestehend aus Midodrin, Cortison, Prednison, Trimipramin, Venlafaxin, anabolen Steroiden, Antidepressiva, Arzneistoffen gegen Fettleibigkeit, CETP-Inhibitoren, pflanzlichen Präparaten, Immunsuppressiva, Mineralocorticoiden, NSAR/Coxibe, Serotonergika, Stimulantien, Sulfonylharnstoffen und sympathomimetischen Aminen ausgewählt ist.

8. Verbindung zur Verwendung nach einem Anspruch 7, wobei der Blutdruck des Individuums vor und nach Verabreichung des Cilnidipin und des blutdrucksteigernden Mittels im Wesentlichen gleich ist.

9. Verbindung zur Verwendung der Ansprüche 1 bis 8, wobei die Knochendichte des Individuums nicht abnimmt.

10. Verbindung zur Verwendung nach einem der Ansprüche 1 bis 9, wobei bei dem Individuum eine verringerte Knochendichte zur Behandlung identifiziert oder diagnostiziert wurde, gegebenenfalls wobei das Individuum, bei dem eine verringerte Knochendichte identifiziert oder diagnostiziert wurde, an Osteoporose leidet, wobei das Individuum weiblich ist.

11. Verbindung zur Verwendung nach Anspruch 1 bis 10, wobei bei dem Individuum eine verringerte Nierenfunktion zur Behandlung identifiziert oder diagnostiziert wurde, gegebenenfalls wobei die Nierenfunktion des Patienten nach der Behandlung nicht herabgesetzt ist.

12. Verbindung zur Verwendung nach einem der Ansprüche 1 bis 11, wobei die Dosierung von Cilnidipin bei etwa 0,005 mg/kg bis etwa 2 mg/kg liegt, gegebenenfalls wobei die Dosierung bei etwa 0,06 mg/kg bis etwa 0,3 mg/kg liegt, gegebenenfalls wobei die Dosierung bei etwa 0,1 mg/kg bis etwa 0,18 mg/kg liegt.

13. Verbindung zur Verwendung nach einem der Ansprüche 1 bis 12, wobei das Cilnidipin oral, parenteral, transdermal, durch Inhalation, intranasal, sublingual, neuraxial oder okular verabreicht wird.

14. Verbindung zur Verwendung nach einem der Ansprüche 1 bis 13, wobei zwischen den einzelnen Verabreichungen des Cilnidipin jeweils mindestens etwa 24 Stunden, gegebenenfalls mindestens etwa 48 Stunden, gegebenenfalls mindestens etwa 72 Stunden, gegebenenfalls mindestens etwa 1 Woche liegen bzw. liegt.

15. Verbindung zur Verwendung nach einem der Ansprüche 1 bis 14, wobei dem Individuum kein Antioxidans verabreicht wird, gegebenenfalls wobei das Antioxidans aus der Gruppe bestehend aus einer Hydralazinverbindung, einem Glutathion, Vitamin C, Cystein, β-Carotin, einem Ubichinon, einem Ubichinol-10, einem Tocopherol, Coenzym Q oder einem Gemisch davon ausgewählt ist.

## Revendications

1. Composé qui est la cilnidipine ou un sel pharmaceutiquement acceptable de celle-ci, pour une utilisation dans le traitement du syndrome de Raynaud chez un sujet qui en a besoin.

2. Composé pour utilisation selon la revendication 1, dans lequel le syndrome de Raynaud est choisi dans le groupe constitué du : syndrome de Raynaud primaire ; syndrome de Raynaud secondaire ; syndrome de Raynaud du mamelon, du nez, de l'oreille, du pénis, de la langue, et/ou de toute région circulatoire alaire, éventuellement dans lequel le syndrome de Raynaud est un syndrome de Raynaud secondaire.

3. Composé pour utilisation selon la revendication 1 ou 2, dans lequel le sujet est également diagnostiqué de l'hypertension ; et dans lequel, après administration de cilnidipine au sujet, la pression artérielle du sujet est réduite, éventuellement dans lequel la pression artérielle systolique du sujet est réduite de plus de 10 mm Hg.

4. Composé pour utilisation selon l'une quelconque des revendications 1 à 3, dans lequel le sujet est traité pour un lupus, une sclérodermie, une sclérodermie avec maladie pulmonaire interstitielle, une fibrose pulmonaire idiopathique, une hypertension pulmonaire primaire, une polyarthrite rhumatoïde, une athérosclérose, une cryoglobulinémie, une polycythémie, une dermatomyosite, une polymyosite, un syndrome de Sjögren ou toute combinaison de ceux-ci, éventuellement dans lequel le sujet est traité pour une sclérodermie, éventuellement dans lequel le sujet est traité pour une sclérodermie avec maladie pulmonaire interstitielle, éventuellement dans lequel le sujet est traité pour un lupus, éventuellement dans lequel le sujet est traité pour une polyarthrite rhumatoïde, éventuellement dans lequel le sujet est traité pour un syndrome de Sjögren, éventuellement dans lequel le sujet est traité pour une fibrose pulmonaire idiopathique.

5. Composé pour utilisation selon la revendication 4, comprenant en outre un agent choisi dans le groupe constitué de : un inhibiteur de la calcineurine, le cyclophosphamide, le nintedanib, le méthotrexate, le mycophénolate, un glucocorticoïde, un médicament anti-inflammatoire non stéroïdien, la D-pénicillamine, un diurétique, l'oméprazole, le bosentan, l'époprosténol, l'énalapril, le lisinopril, le captopril ou toute combinaison de ceux-ci, comprenant éventuellement en outre le nintedanib, comprenant éventuellement en outre un inhibiteur de la calcineurine, un médicament anti-inflammatoire non stéroïdien ou les deux, éventuellement dans lequel l'inhibiteur de la calcineurine est une cyclosporine et éventuellement, dans lequel le médicament anti-inflammatoire non stéroïdien est l'aspirine ou comprenant en outre un agent choisi dans le groupe constitué de : un médicament antipaludique, un médicament anti-inflammatoire non stéroïdien, le bélimumab, un corticostéroïde, un immunosuppresseur ou toute combinaison de ceux-ci ou comprenant en outre un agent choisi dans le groupe constitué de : le méthotrexate, la sulfasalazine, un médicament anti-inflammatoire non stéroïdien, un corticostéroïde, un produit biologique ou toute combinaison de ceux-ci, ou comprenant en outre un agent choisi dans le groupe constitué de : le plaquénil, un médicament antipaludique, l'evoxac, la céviméline, l'infliximab ou toute combinaison de ceux-ci, ou comprenant en outre un agent choisi dans le groupe constitué de : le nintedanib, la pirfénidone ou toute combinaison de ceux-ci.

6. Composé pour l'utilisation selon l'une quelconque des revendications 1 à 5, comprenant au moins un bloqueur des canaux calciques supplémentaire, éventuellement dans lequel l'au moins un bloqueur des canaux calciques supplémentaire est choisi dans le groupe constitué de : l'amlodipine, la nifédipine, la nicardipine, la nimodipine, le vérapamil, le diltiazem, la félodipine, l'isradipine, la nisoldipine et la nitrendipine.

7. Composé pour utilisation selon l'une quelconque des revendications 1 à 6, comprenant en outre une quantité thérapeutiquement efficace d'un agent qui augmente la pression sanguine, éventuellement dans lequel l'agent qui augmente la pression sanguine est choisi dans le groupe constitué de : la midodrine, la cortisone, la prednisone, la trimipramine, la venlafaxine, les stéroïdes anabolisants, les antidépresseurs, les médicaments anti-obésité, les inhibiteurs de CETP, les préparations à base de plantes, les immunosuppresseurs, les minéralocorticoïdes, les AINS/coxibs, les sérotonergiques, les stimulants, les sulfonylurées et les amines sympathomimétiques.

8. Composé pour utilisation selon la revendication 7, dans lequel la pression sanguine du sujet avant et après administration de la cilnidipine et de l'agent qui augmente la pression sanguine est sensiblement la même.

9. Composé pour l'utilisation selon les revendications 1 à 8, dans lequel la densité osseuse du sujet ne diminue pas.

10. Composé pour l'utilisation selon l'une quelconque des revendications 1 à 9, dans lequel le sujet est identifié ou diagnostiqué comme ayant une densité osseuse réduite pour le traitement, éventuellement dans lequel le sujet identifié ou diagnostiqué comme ayant une densité osseuse réduite est atteint d'ostéoporose, éventuellement dans lequel le sujet est une femme.

11. Composé pour l'utilisation selon les revendications 1 à 10, dans lequel le sujet est identifié ou diagnostiqué comme ayant une fonction rénale réduite pour le traitement, éventuellement dans lequel la fonction rénale du patient n'est pas réduite après le traitement.

12. Composé pour l'utilisation selon l'une quelconque des revendications 1 à 11, dans lequel la dose de cilnidipine est d'environ 0,005 mg/kg à environ 2 mg/kg, éventuellement dans lequel la dose est d'environ 0,06 mg/kg à environ 0,3 mg/kg, éventuellement dans lequel la dose est d'environ 0,1 mg/kg à environ 0,18 mg/kg.

13. Composé pour l'utilisation selon l'une quelconque des revendications 1 à 12, dans lequel la cilnidipine est administrée par voie orale, parentérale, transdermique, par inhalation, par voie intranasale, sublinguale, neuraxiale ou oculaire.

14. Composé pour l'utilisation selon l'une quelconque des revendications 1 à 13, dans lequel chaque administration de la cilnidipine est séparée d'au moins environ 24 heures, éventuellement d'au moins environ 48 heures, éventuellement d'au moins environ 72 heures, éventuellement d'au moins environ 1 semaine.

15. Composé pour l'utilisation selon l'une quelconque des revendications 1 à 14, dans lequel un antioxydant n'est pas administré au sujet, éventuellement dans lequel l'antioxydant est choisi dans le groupe constitué d'un composé d'hydralazine, du glutathion, de la vitamine C, de la cystéine, du β-carotène, de l'ubiquinone, de l'ubiquinol-10, du tocophérol, de la coenzyme Q ou d'un mélange de ceux-ci.
